# EUROPEAN PATENT SPECIFICATION

(11) **EP 1 737 838 B1**
(45) Date of publication and mention of the grant of the patent: **21.05.2008**
(21) Application number: 05729797.0
(22) Date of filing: 04.04.2005
(51) Int. Cl.: C07D 401/02

(54) **SUBSTITUTED 4-ALKYL- AND 4-ALKANOYL-PIPERIDINE DERIVATIVES AND THEIR USE AS NEUROKININ ANTAGONISTS**
SUBSTITUIERTE 4-ALKYL- UND 4-ALKANOYLPIPERIDINDERIVATE UND DEREN VERWENDUNG ALS NEUROKININANTAGONISTEN
DERIVES 4-ALKYL-ET 4-ALKANOYL-PIPERIDINES ET UTILISATIONS DE CES DERNIERS EN TANT QU'ANTAGONISTES DE LA NEUROKININE

(30) Priority: 08.04.2004 EP 04101467
(43) Date of publication of application: 03.01.2007
(73) Proprietor: Janssen Pharmaceutica NV, 2340 Beerse (BE)
(72) Inventor: JANSSENS, Frans Eduard, Janssen Pharmaceutica N.V., B-2340 Beerse (BE); SOMMEN, François Maria, Janssen Pharmaceutica N.V., B-2340 Beerse (BE); DE BOECK, B.C.A.G., Janssen Pharmaceutica N.V., B-2340Beerse (BE); LEENAERTS, Joseph E., Janssen Pharmaceutica N.V., B-2340 Beerse (BE)
(74) Representative: Daelemans, Frank F.R.
(86) International application number: PCT/EP2005/051509
(87) International publication number: WO 2005/097774

(56) References cited:
- WO-A-02/32867
- WO-A-97/16440
- WO-A-97/24356
- WO-A-02/062784
- WO-A-20/04056805

## Description

### Field of the Invention

This invention concerns substituted 4-alkyl- and 4-alkanoyl-piperidine derivatives having neurokinin antagonistic activity, in particular NK₁ antagonistic activity and a combined NK₁/NK₃ antagonistic activity, compositions comprising them and their use as a medicine, in particular for the treatment and/or prophylaxis of schizophrenia, emesis, anxiety and depression, irritable bowel syndrome (IBS), circadian rhythm disturbances, pre-eclampsia, nociception, pain, in particular visceral and neuropathic pain, pancreatitis, neurogenic inflammation, asthma, chronic obstructive pulmonary disease (COPD) and micturition disorders such as urinary incontinence.

### Background of The Invention

Tachykinins belong to a family of short peptides that are widely distributed in the mammalian central and peripheral nervous system (Bertrand and Geppetti, Trends Pharmacol. Sci. 17:255-259 (1996); Lundberg, Can. J. Physiol. Pharmacol. 73:908-914 (1995) ; Maggi, Gen. Pharmacol. 26:911-944 (1995) ; Regoli et al., Pharmacol. Rev. 46 (1994)). They share the common C-terminal sequence Phe-Xaa-Gly-Leu-Mat-NH₂. Tachykinins released from peripheral sensory nerve endings are believed to be involved in neurogenic inflammation. In the spinal cord/central nervous system, tachykinins may play a role in pain transrnission/perception and in some autonomic reflexes and behaviors. The three major tachykinins are Substance P (SP), Neurokinin A (NKA) and Neurokinin B (NKB) with preferential affinity for three distinct neurokinin receptor subtypes, termed NK₁, NK₂, and NK₃, respectively. However, functional studies on cloned receptors suggest strong functional cross-interaction between the 3 tachykinins and their corresponding neurokinin receptors (Maggi and Schwartz, Trends Pharmacol. Sci. 18: 351-355 (1997)).

Species differences in structure of NK₁ receptors are responsible for species-related potency differences of NK₁ antagonists (Maggi, Gen. Pharmacol. 26:911-944 (1995) ; Regoli et al., Pharmacol. Rev. 46(4):551-599 (1994)). The human NK₁ receptor closely resembles the NK₁ receptor of guinea-pigs and gerbils but differs markedly from the NK₁ receptor of rodents. The development of neurokinin antagonists has led to date to a series of peptide compounds of which might be anticipated that they are metabolically too labile to be employed as pharmaceutically active substances (Longmore J. et al., DN&P 8(1):5-23 (1995)).

The tachykinins are involved in schizophrenia, depression, (stress-related) anxiety states, emesis, inflammatory responses, smooth muscle contraction and pain perception. Neurokinin antagonists are in development for indications such as emesis, anxiety and depression, irritable bowel syndrome (IBS), circadian rhythm disturbances, visceral pain, neurogenic inflammation, asthma, micturition disorders, and nociception. In particular, NK₁ antagonists have a high therapeutic potential in emesis and depression and NK₂ antagonists have a high therapeutic potential in asthma treatments. NK₃ antagonists seem to play a role in the treatment of pain/inflammation (Giardina, G. et al. Exp. Opin. Ther. Patents, 10(6): 939-960 (2000)) and schizophrenia.

### Schizophrenia

The NK₃ antagonist SR142801 (Sanofi) was recently shown to have antipsychotic activity in schizophrenic patients without affecting negative symptoms (Arvantis, L. ACNP Meeting, December 2001). Activation of NK₁ receptors causes anxiety, stressfull events evoke elevated substance **P** (SP) plasma levels and NK₁ antagonists are reported to be anxiolytic in several animal models. The NK₁ antagonist from Merck, MK-869 shows antidepressant effects in major depression, but data were not conclusive due to a high placebo response rate. Moreover, the NK₁ antagonist from Glaxo-Welcome (S)-GR205,171 was shown to enhance dopamine release in the frontal cortex but not in the striatum (Lejeune et al. Soc. Neurosci., November 2001). It is therefore hypothesized that NK₃ antagonism in combination with NK₁ antagonism would be beneficial against both positive and negative symptoms of schizophrenia.

### Anxiety and depression

Depression is one of the most common affective disorders of modem society with a high and still increasing prevalence, particularly in the younger members of the population. The life time prevalence rates of Major depression (MDD, DSM-IV) is currently estimated to be 10-25 % for women and 5-12 % for men, whereby in about 25 % of patients the life time MDD is recurrent, without full inter-episode recovery and superimposed on dysthymic disorder. There is a high co-morbidity of depression with other mental disorders and, particularly in younger population high association with drug and alcohol abuse. In the view of the fact that depression primarily affects the population between 18-44 years of age e.g. the most productive population, it is obvious that it imposes a high burden on individuals, families and the whole society.

Among all therapeutic possibilities, the therapy with antidepressants is incontestably the most effective. A large number of antidepressants have been developed and introduced to the market in the course of the last 40 years. Nevertheless, none of the current antidepressants fulfill all criteria of an ideal drug (high therapeutic and prophylactic efficacy, rapid onset of action, completely satisfactory short- and long-term safety, simple and favourable pharmacokinetics) or is without side effects which in one or the other way limits their use in all groups and subgroups of depressed patients.

Since no treatment of the cause of depression exists at present, nor appears imminent, and no antidepressant is effective in more than 60-70 % of patients; the development of a new antidepressant which may circumvent any of the disadvantages of the available drugs is justified.

Several findings indicate involvement of SP in stress-related anxiety states. Central injection of SP induces a cardiovascular response resembling the classical "fight or flight" reaction characterised physiologically by vascular dilatation in skeletal muscles and decrease of mesenteric and renal blood flow. This cardiovascular reaction is accompanied by a behavioural response observed in rodents after noxious stimuli or stress (Culman and Unger, Can. J. Physiol. Pharmacol. 73:885-891 (1995)). In mice, centrally administered NK₁ agonists and antagonists are anxiogenic and anxiolytic, respectively (Teixeira et al., Eur. J. Pharmacol. 311:7-14 (1996)). The ability of NK₁ antagonists to inhibit thumping induced by SP (or by electric shock; Ballard et al., Trends Pharmacol. Sci. 17:255-259 (2001)) might correspond to this antidepressant / anxiolytic activity, since in gerbils thumping plays a role as an alerting or warning signal to conspecifics.

The NK₁ receptor is widely distributed throughout the limbic system and fear-processing pathways of the brain, including the amygdala, hippocampus, septum, hypothalamus, and periaqueductal grey. Additionally, substance **P** is released centrally in response to traumatic or noxious stimuli and substance P-associated neuro-transmission may contribute to or be involved in anxiety, fear, and the emotional disturbances that accompany affective disorders such as depression and anxiety. In support of this view, changes in substance **P** content in discrete brain regions can be observed in response to stressful stimuli (Brodin et al., Neuropeptides 26:253-260 (1994)).

Central injection of substance P mimetics (agonists) induces a range of defensive behavioural and cardiovascular alterations including conditioned place aversion (Elliott, Exp. Brain. Res. 73:354-356 (1988)), potentiated acoustic startle response (Krase et al., Behav. Brain. Res. 63:81-88 (1994)), distress vocalisations, escape behaviour (Kramer et al., Science 281:1640-1645 (1998)) and anxiety on the elevated plus maze (Aguiar and Brandao, Physiol. Behav. 60:1183-1186 (1996)). These compounds did not modify motor performance and co-ordination on the rotarod apparatus or ambulation in an activity cage. Down-regulation of substance **P** biosynthesis occurs in response to the administration of known anxiolytic and antidepressant drugs (Brodin et al., Neuropeptides 26:253-260 (1994) ; Shirayama et al., Brain. Res. 739:70-78 (1996)). Similarly, a centrally administered NK₁ agonist-induced vocalisation response in guinea-pigs can be antagonised by antidepressants such as imipramine and fluoxetine as well as L-733,060, an NK₁ antagonist. These studies provide evidence suggesting that blockade of central NK₁ receptors may inhibit psychological stress in a manner resembling antidepressants and anxiolytics (Rupniak and Kramer, Trends Pharmacol. Sci. 20:1-12 (1999)), but without the side effects of present medications.

### Emesis

Nausea and vomiting are among the most distressing side effects of cancer chemotherapy. These reduce the quality of life and may cause patients to delay or refuse, potentially curative drugs (Kris et al., J. Clin. Oncol., 3:1379-1384 (1985)). The incidence, intensity and pattern of emesis is determined by different factors, such as the chemotherapeutic agent, dosage and route of administration. Typically, early or acute emesis starts within the first 4 h after chemotherapy administration, reaching a peak between 4 h and 10 h, and decreases by 12 to 24 h. Delayed emesis (developing after 24 h and continuing until 3-5 days post chemotherapy) is observed with most 'high-emetogenic' chemotherapeutic drugs (level 4 and 5 according to Hesketh et al., J. Clin. Oncol. 15:103 (1997)). In humans, these 'high-emetogenic' anti-cancer treatments, including cis-platinum, induce acute emesis in > 98% and delayed emesis in 60-90% of cancer patients.

Animal models of chemotherapy such as cisplatin-induced emesis in ferrets (Rudd and Naylor, Neuropharmacology 33:1607-1608 (1994) ; Naylor and Rudd, Cancer. Surv 21:117-135 (1996)) have successfully predicted the clinical efficacy of the 5-HT₃ receptor antagonists. Although this discovery led to a successful therapy for the treatment of chemotherapy- and radiation-induced sickness in cancer patients, 5-HT₃ antagonists such as ondansetron and granisetron (either or not associated with dexamethasone) are effective in the control of the acute emetic phase (the first 24 h) but can only reduce the development of delayed emesis (> 24 h) with poor efficacy (De Mulder et al., Annuals of Internal Medicine 113:834-840 (1990); Roila, Oncology 50:163-167 (1993)). Despite these currently most effective treatments for the prevention of both acute and delayed emesis, still 50% of patients suffer from delayed vomiting and/or nausea (Antiemetic Subcommittee, Annals Oncol. 9:811-819 (1998)).

In contrast to 5-HT₃ antagonists, NK₁ antagonists such as CP-99,994 (Piedimonte et al., L. Pharmacol. Exp. Ther. 266:270-273 (1993)) and aprepitant (also known as MK-869 or L-754,030 ; Kramer et al., Science 281:1640-1645 (1998) ; Rupniak and Kranser, Trends Pharmacol. Sci. 20:1-12 (1999)) have now been shown to inhibit not only the acute but also the delayed phase of cisplatin-induced emesis in animals (Rudd et al., Br. J. Pharmacol. 119:931-936 (1996) ; Tattersall et al., Neuropharmacology 39:652-663 (2000)). NK₁ antagonists have also been demonstrated to reduce 'delayed' emesis in man in the absence of concomitant therapy (Cocquyt et al., Eur. J. Cancer 37:835-842 (2001); Navari et al., N. Engl. L. Med. 340:190-195 (1999)). When administered together with dexamethasone and 5-HT₃ antagonists, moreover, NK₁ antagonists (such as MK-869 and CJ-11,974, also known as Ezlopitant) have been shown to produce additional effects in the prevention of acute emesis (Campos et al., J. Clin. Oncol. 19:1759-1767 (2001) ; Hesketh et al., Clin. Oncol. 17:338-343 (1999)).

Central neurokinin NK₁ receptors play a major role in the regulation of emesis. NK₁ antagonists are active against a wide variety of emetic stimuli (Watson et al., Br. J. Pharmacol. 115:84-94 (1995) ; Tattersall et al., Neuropharmacol. 35:1121-1129 (1996) ; Megens et al., J. Pharmacol. Exp. Ther. 302:696-709 (2002)). The compounds are suggested to act by blocking central NK₁-receptors in the nucleus tractus solitarius. Apart from NK₁ antagonism, CNS penetration is thus a prerequisite for the antiemetic activity of these compounds. Loperamide-mduced emesis in ferrets can be used as a fast and reliable screening model for the antiemetic activity of NK₁ antagonists. Further evaluation of their therapeutic value in the treatment of both the acute and the delayed phases of cisplatin-induced emesis has been demonstrated in the established ferret model (Rudd et al., Br. J. Pharmacol. 119:931-936 (1994)). This model studies both 'acute' and 'delayed' emesis after cisplatin and has been validated in terms of its sensitivity to 5-HT₃ receptor antagonists, glucocorticoids (Sam et al., Eur. J. Pharmacol. 417:231-237 (2001)) and other pharmacological challenges. It is unlikely that any future anti-emetic would find clinical acceptance unless successfully treating both the 'acute' and 'delayed' phases of emesis.

### Visceral pain and Irritable bowel syndrome (IBS)

Visceral sensation refers to all sensory information that originates in the viscera (heart, lungs, GI tract, hepatobiliary tract and urogenital tract), and is transmitted to the central nervous system resulting in conscious perception. Both the vagal nerve via the nodose ganglion and the primary sympathetic afferent nerves via dorsal root ganglias (DRG) and second order neurons in the dorsal horn serve as the initial pathways along which visceral sensory information is conveyed to the brain stem and to the viscero-somatic cortex. Visceral pain may be caused by neoplastic processes (e.g. pancreas cancer), inflammation (e.g. cholecystitis, peritonitis), ischemia and mechanical obstruction (e.g. urether stone).

The mainstay of medical treatment for visceral pain linked to organic disorders (in casu cancer of the viscera) still focuses on opiates.

Recent evidence suggests that non-organic visceral disorders such as irritable bowel syndrome (IBS), non-cardiac chest pain (NCCP) and chronic pelvic pain may originate from a state of "visceral hyperalgia". The latter is defined as a condition in which physiological, non-painful visceral stimuli (e.g. gut distension) lead to conscious perception of pain due to a decreased threshold for pain. Visceral hyperalgesia may reflect a state of a permanent, post-inflammatory resetting of the threshold for membrane depolarization at neuronal synapses within visceral sensory pathways. The initial inflammation may occur at the periphery (e.g. infectuous gastroenteritis) or at the site of visceral sensory information integration (neurogenic inflammation in the dorsal horn). Both SP and calcitonin gene-related peptide (CGRP) have been shown to act as pro-inflammatory neuropeptides in neurogenic inflammation.

Visceral hyperalgesia is currently considered as one of the prime targets for drug development aimed at treating functional bowel diseases, which occur in 15 to 25% of the western population. They constitute an enormous socio-economic problem in terms of medical care costs, prescription costs and absenteism. Current treatment options include anti-spasmodics (IBS and NCCP), promotility agents (e.g. tegasorod in constipation-IBS), laxatives (constipation-IBS), and loperamide (diarrhea-IBS), amongst others. None of these approaches has been shown to be very effective, particularly in treating pain. Low dose tricyclic antidepressants and SSRIs are used to treat visceral hyperalgesia in pain-predominant IBS, but both classes of compounds may have considerable effects on colonic transit. Ongoing research in this field has identified a considerable number of molecular targets that could serve for drug development in visceral hyperalgesia. These include NK receptors, the CGRP receptor, 5-HT₃ receptors, glutamate receptors, and the kappa opioid receptor. Ideally, a "visceral analgesic compound" should block heightened sensory transfer from the viscera to the CNS without affecting the normal physiological homeostasis of the GI tract with regards to propulsive motor activity, absorption and secretion, and sensation. There is compelling evidence linking tachykinin to visceral nociceptive signalling. A number of pre-clinical publications on the role of NK₁, NK₂ and NK₃ receptors in visceral pain and visceral hyperalgesia indicate a discrepancy between the implication of NK₁, NK₂ and NK₃ receptors in the different inflammation hypersensitivity rodent models. Recently, Kamp et al., J. Pharmacol. Exp. Ther.299:105-113 (2001) suggested that a combined neurokinin receptor antagonist could be more active than a selective neurokinin receptor antagonist Substance P and NK₁, NK₂ and NK₃ receptors are elevated in clinical pain states, including visceral pain states (Lee et al., Gastroenterol. 118: A846 (2000)). Given the recent failures of NK₁ receptor antagonists as an analgesic in human pain trials (Goldstein et al., Clin. Pharm. Ther. 67:419-426 (2000)), combinations of antagonists may be necessary to have a significant clinical effect NK₃ receptor antagonists are anti-hyperalgesic (Julia et al., Gastroenterol. 116:1124-1131 (1999)); J. Pharmacol. Exp. Ther.299:105-113 (2001)). Recently, the involvement of NK₁ and NK₃ receptors but not NK₂ receptors at spinal level was demonstrated in visceral hypersensitivity mediated by nociceptive and non-nociceptive afferent inputs (Gaudreau & Ploudre, Neurosci. Lett. 351:59-62 (2003). Combining the NK₁₋₂₋₃ antagonistic activity could therefore represent an interesting therapeutic target for the development of novel treatments for visceral hyperalgesia.

A reasonable number of pre-clinical publications over the role of NK₁ receptors in visceral pain has been published. Using NK₁ receptor knockout mice and NK₁ antagonists in animal models, different groups have demonstrated the important role played by the NK₁ receptor in hyperalgesia and visceral pain. The distribution of NK₁ receptors and substance P favours a major role in visceral rather than in somatic pain. Indeed more than 80% of visceral primary afferent contain substance P compared with only 25% skin afferents. NK₁ receptors are also involved in gastrointestinal motility (Tonini et al., Gastroenterol. 120:938-945 (2001) ; Okano et al., J. Pharmacol. Exp. Ther. 298:559-564 (2001)). Because of this dual role in both gastrointestinal motility and in nociception, NK₁ antagonists are considered to have potential to ameliorate symptoms in IBS patients.

### Urinary incontinence

Urge urinary incontinence is caused by urinary bladder or detrusor hyperreflexia ("irritable bladder"). This hyperreflexia relates to hyperexcitability of bladder sensory afferent C-fibers projecting to the spinal cord. The origin of C-fiber hyperexcitability is multifactorial but occurs for example after bladder infection and chronic distention of the bladder wall (eg. benign prostate hypertrophy, BPH). Hence, treatment should be aimed at decreasing neuronal hyperexcitability. Intravesical instillation of vanilloids (eg. capsaicin) results in a long-term beneficial effect on detrusor hyperreflexia refractory to conventional treatment with anticholinergic drugs. Analogous to animal studies, the effect of vanilloids is mediated through a neurotoxic effect on sensory nerve terminals. In human bladder, subendothelial sensory nerves contain tachykinins, which drive detrusor hyperexcitability. The NK receptors involved in this effect are peripheral NK₂ receptors and to a lesser extent, also NK₁ receptors. The latter are claimed to play a role in bladder hyperreflexia at the level of the spinal cord As a consequence, a centrally acting NK₁/penpherally acting NK₂ antagonist is preferred for the treatment of detrusor hyperexcitability. Interestingly, activation of NK₂ receptors increases aromatase activity in Sertoli cells. NK₂ receptor antagonists reduce serum testosterone levels in mice, and this may be of therapeutic importance in BPH.

### Background prior art

Compounds containing a 1-piperidin-4-yl-piperazinyl moiety were published in WO 97/16440-A1, published May 9, 1997 by Janssen Pharmaceutica N.V. for use as substance P antagonists, in WO 02/32867, published April 25, 2002 by Glaxo Group Ltd. for their special advantages as neurokinin antagonists (more specifically were disclosed 4-piperazin-1-yl-piperidine-1-carboxylic acid amide derivatives), in WO 01/30348-A1, published May 03, 2001 by Janssen Pharmaceutica N.V., for use as substance P antagonists for influencing the circadian timing system, and in WO 02/062784-A1, published August 15, 2002 by Hoffmann-La Roche AG for use as neurokinin 1 antagonists.

The compounds of the present invention differ from the compounds of the prior art in the substitution of the piperazinyl/imidazolidinyl moiety, as well as in their improved ability as potent, orally and centrally active neurokinin antagonists with therapeutic value, especially for the treatment and/or prophylaxis of schizophrenia, emesis, anxiety and depression, irritable bowel syndrome (IBS), circadian rhythm disturbances, pre-eclampsia, nociception, pain, in particular visceral and neuropathic pain, pancreatitis, neurogenic inflammation, asthma, chronic obstructive pulmonary disease (COPD) and micturition disorders such as urinary incontinence.

### Description of the Invention

The present invention relates to novel 4-alkyl- and 4-alkanoyl-piperidine derivatives according to the general Formula (I) 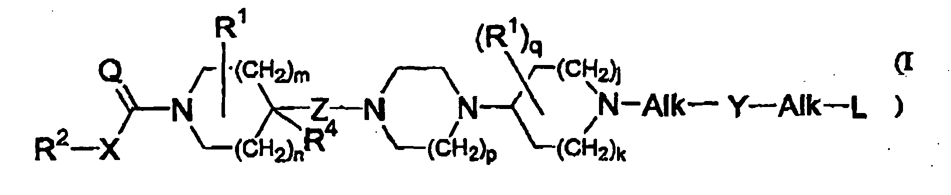 the pharmaceutically acceptable acid or base addition salts thereof, the stereochemically isomeric forms thereof, the *N*-oxide form thereof and prodrugs thereof, wherein :
- n: is an integer, equal to 1;
- m: is an integer, equal to 1;
- each R¹: independently from each other, is selected from the group of Ar¹, Ar¹-alkyl and di(Ar¹)-alkyl;
- R⁴: is selected from the group of hydrogen, hydroxy and alkyloxy;
- Z: is a bivalent radical-(CH₂)ᵣ-, wherein r is an integer equal to 1, 2, 3, 4 or 5 and wherein one radical -CH₂- is optionally replaced by a >C=O radical ; or
- R⁴ and Z: are taken together to form a trivalent radical =CH-(CH₂)ᵣ₋₁-, wherein r is an integer equal to 2, 3, 4 or 5 and wherein one radical -CH₂- is optionally replaced by a >C=O radical ;
- p: is an integer equal to 1;
- Q: is O or NR³;
- X: is a covalent bond or a bivalent radical of formula -O-, -S- or -NR³-;
- each R³: independently from each other, is hydrogen or alkyl;
- R²: is alkyl, Ar², Ar²-alkyl, Het¹ or Het¹-alkyl;
- q: is an integer, equal to 0 or 1 ;
- j: is an integer, equal to 1;
- k: is an integer, equal to 0, 1 ;
- Y: is a covalent bond or a bivalent radical of formula >C(=O) or -SO₂- ;
- each Alk: represents, independently from each other, a covalent bond ; a bivalent straight or branched, saturated or unsaturated hydrocarbon radical having from 1 to 6 carbon atoms ; or a cyclic saturated or unsaturated hydrocarbon radical having from 3 to 6 carbon atoms; each radical optionally substituted on one or more carbon atoms with one or more alkyl, phenyl, halo, cyano, hydroxy, formyl and amino radicals ;
- L: is selected from the group of hydrogen, alkyl, alkyloxy, Ar³-oxy, alkyloxycarbonyl, mono- and di(alkyl)amino, mono- and di(Ar³)amino, mono-and di(alkyloxycarbonyl)amino, Ar³, Ar³-carbonyl, Het² and Het²-carbonyl ;
- Ar¹: is phenyl, optionally substituted with 1, 2 or 3 substituents, each independently from each other, selected from the group of halo, alkyl, cyano, aminocarbonyl and alkyloxy ;
- Ar²: is naphthalenyl or phenyl, each optionally substituted with 1, 2 or 3 substituents, each independently from each other, selected from the group of halo, nitro, amino, mono- and di(alkyl)amino, cyano, alkyl, hydroxy, alkyloxy, carboxyl, alkyloxycarbonyl, aminocarbonyl and mono- and di(alkyl)aminocarbonyl ;
- Ar³: is naphthalenyl or phenyl, optionally substituted with 1, 2 or 3 substituents, each independently from each other, selected from the group of alkyloxy, alkyl, halo, hydroxy, pyridinyl, morpholinyl, pyrrolidinyl, imidazo[1,2-*a*]pyridinyl, morpholinylcarbonyl, pyrrolidinylcarbonyl, amino and cyano ;
- Het¹: is a monocyclic heterocyclic radical selected from the group of pyrrolyl, pyrazolyl, imidazolyl, furanyl, thienyl, oxazolyl, isoxazolyl, thiazolyl, thiadiazolyl, isothiazolyl, pyridinyl, pyrimidinyl, pyrazinyl and pyridazinyl ; or a bicyclic heterocyclic radical selected from the group of quinolinyl, quinoxalinyl, indolyl, benzimidazolyl, benzoxazolyl, benzisoxazolyl, benzothiazolyl, benzisothiazolyl, benzofuranyl and benzothienyl ; each heterocyclic radical may optionally be substituted on any atom by one or more radicals selected from the group of halo and alkyl ;
- Het²: is a monocyclic heterocyclic radical selected from the group of pyrrolidinyl, dioxolyl, imidazolidinyl, pyrrazolidinyl, piperidinyl, morpholinyl, dithianyl, thiomorpholinyl, piperazinyl, imidazolidinyl, tetrahydrofuranyl, 2H-pyrrolyl, pyrrolinyl imidazolinyl, pyrrazolinyl, pyrrolyl, imidazolyl, pyrazolyl, triazolyl, furanyl, thienyl, oxazolyl, isoxazolyl, thiazolyl, thiadiazolyl, isothiazolyl, pyridinyl, pyrimidinyl, pyrazinyl, pyridazinyl and triazinyl ; or a bicyclic heterocyclic radical selected from the group of benzopiperidinyl, quinolinyl, quinoxalinyl, indolyl, isoindolyl, chromenyl, benzimidazolyl, imidazo[1,2-*a*]pyridinyl, benzoxazolyl, benzisoxazolyl, benzothiazolyl, benzisothiazolyl, benzofuranyl and benzothienyl; each heterocyclic radical may optionally be substituted on any atom by one or more radicals selected from the group of Ar¹, Ar¹ alkyl, halo, hydroxy, alkyl, piperidinyl, pyrrolyl, thienyl, oxo, alkyloxy, alkyloxyalkyl and alkyloxycarbonyl; and
- alkyl: is a straight or branched saturated hydrocarbon radical having from 1 to 6 carbon atoms or a cyclic saturated hydrocarbon radicals having from 3 to 6 carbon atoms ; optionally substituted on one or more carbon atoms with one or more radicals selected from the group of phenyl, halo, cyano, oxo, hydroxy, formyl and amino radicals.

More in particular, the invention relates to a compound according to the general Formula (I), the pharmaceutically acceptable acid or base addition salts thereof, the stereochemically isomeric forms thereof the *N*-oxide form thereof and a prodrug thereof, wherein R¹ is Ar¹ methyl and attached to the 2-position or R¹ is Ar¹ and attached to the 3-position, as exemplified in either of the following formulas for compounds according to Formula (I) wherein m and n are equal to 1 and Ar is an unsubstituted phenyl. Preferably, Ar¹ is an unsubstituted phenyl radical and Ar¹ methyl is an unsubstituted benzyl radical

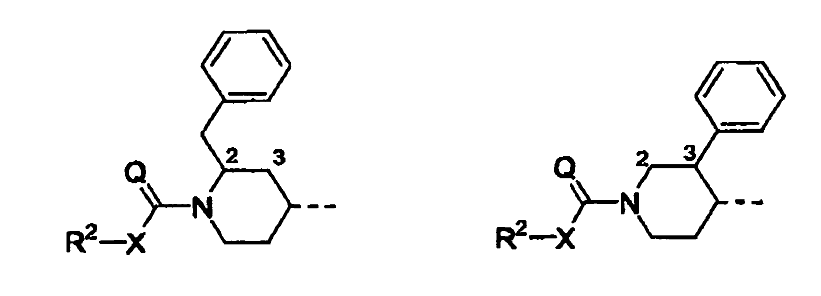

More in particular, the invention relates to a compound according to the general Formula (I), the pharmaceutically acceptable acid or base addition salts thereof, the stereochemically isomeric forms thereof, the *N-*oxide form thereof and a prodrug thereof, wherein the R²-X-C(=Q)- moiety is 3,5-di-(trifluoromethyl)phenylcarbonyl.

More in particular, the invention relates to a compound according to the general Formula (I), the pharmaceutically acceptable acid or base addition salts thereof, the stereochemically isomeric forms thereof, the *N*-oxide form thereof and a prodrug thereof, wherein Z is selected from the group of -CH₂-, >C=O, -CH₂CH₂- and -CH₂C(=O)- or wherein Z and R⁴ are taken together to form the trivalent radical =CH-C(=O)-.

More in particular, the invention relates to a compound according to the general Formula (I), the pharmaceutically acceptable acid or base addition salts thereof, the stereochemically isomeric forms thereof, the *N*-oxide form thereof and a prodrug thereof, wherein R⁴ is selected from the group of hydrogen, hydroxy and methoxy.

More in particular, the invention relates to a compound according to the general Formula. (I), the pharmaceutically acceptable acid or base addition salts thereof, the steteochemically isomeric forms thereof, the *N*-oxide form thereof and a prodrug thereof, wherein Alk is a covalent bond, -CH₂-, CH(CH₃)-, -CH(phenyl)-, -CH₂(phenyl)- or -CH₂CH=CH-.

More in particular, the invention relates to a compound according to the general Formula (I), the pharmaceutically acceptable acid or base addition salts thereof, the stereochemically isomeric forms thereof, the *N*-oxide form thereof and a prodrug thereof, wherein L is selected from the group of hydrogen, alkyl, alkyloxy, Ar³-oxy, mono- and di(Ar³)amino, Ar³, Het² and Het² carbonyl and Ar³ and Het² are defined as in Fonoula(I).

More in particular, the invention relates to a compound according to the general Formula (I), the pharmaceutically acceptable acid or base addition salts thereof, the stereochemically isomeric forms thereof, the *N*-oxide form thereof and a prodrug thereof, wherein :
- n: is an integer, equal to 1 ;
- m: is an integer, equal to 1 ;
- R¹: is Ar¹-alkyl ;
- R⁴: is selected from the group of hydrogen, hydroxy or alkyloxy ;
- Z: is a bivalent radical -(CH₂)ᵣ-, wherein r is 1 or 2 and wherein one radical -CH₂- is optionally replaced by a >C=O radical ; or
- R⁴ and Z: are taken together to form a trivalent radical =CH-(CH₂)ᵣ₋₁, wherein r is 2 and wherein one radical -CH₂- is replaced by a >C=O radical ;
- p: is an integer, equal to 1 ;
- Q: is O;
- X: is a covalent bond ;
- R²: is Ar² ;
- q: is an integer, equal to 0 ;
- j: is an integer, equal to 1 ;
- k: is an integer, equal to 0 or 1 ;
- Y: is a covalent bond or a bivalent radical of formula >C(=O) or -SO₂- ;
- each Alk: represents, independently from each other, a covalent bond ; a bivalent straight or branched, saturated or unsaturated hydrocarbon radical having from 1 to 6 carbon atoms ; each radical optionally substituted on one or more carbon atoms with a phenyl radical ;
- L: is selected from the group of hydrogen, alkyl, alkyloxy, Ar³-oxy, mono- and di(Ar³)amino, Ar³ and Het² ;
- Ar¹: is phenyl ;
- Ar²: is phenyl, optionally substituted with 2 alkyl substituents ;
- Ar³: is phenyl, optionally substituted with 1, 2 or 3 substituents, each independently from each other selected from the group of alkyloxy, alkyl, halo and hydroxy ;
- Het²: is a monocyclic heterocyclic radical selected from the group of tetrahydrofuranyl, pyrrolyl, imidazolyl, pyrazolyl, furanyl, thienyl, isoxazolyl, thiadiazolyl, pyridinyl, pyrimidinyl, pyrazinyl and pyridazinyl ; or a bicyclic heterocyclic radical selected from the group of quinolinyl, indolyl, chromenyl and benzimidazolyl ; each heterocyclic radical may optionally be substituted on any atom by one or more radicals selected from the group of Ar¹, halo, alkyl and oxo ; and
- alkyl: is a straight or branched saturated hydrocarbon radical having from 1 to 6 carbon atoms or a cyclic saturated hydrocarbon radicals having from 3 to 6 carbon atoms.

In the framework of this application, alkyl is defined as a monovalent straight or branched saturated hydrocarbon radical having from 1 to 6 carbon atoms, for example methyl, ethyl, propyl, butyl, 1-methylpropyl, 1,1-dimethylethyl, pentyl, hexyl ; alkyl further defines a monovalent cyclic saturated hydrocarbon radical having from 3 to 6 carbon atoms, for example cyclopropyl, methylcyclopropyl, cyclobutyl, cyclopentyl and cyclohexyl. The definition of alkyl also comprises an alkyl radical that is optionally substituted on one or more carbon atoms with one or more phenyl, halo, cyano, oxo, hydroxy, formyl and amino radicals, for example hydroxyalkyl, in particular hydroxymethyl and hydroxyethyl and polyhaloalkyl, in particular difluoromethyl and trifluoromethyl.

In the framework of this application, halo is generic to fluoro, chloro, bromo and iodo.

In the framework of this application, with "compounds according to the invention" is meant a compound according to the general Formula (I), the pharmaceutically acceptable acid or base addition salts thereof, the stereochemically isomeric forms thereof, the *N*-oxide form thereof and a prodrug thereof.

In the framework of this application, especially in the moiety Alk^{a}-Y-Alk^{b} in Formula (I), when two or more consecutive elements of said moiety denote a covalent bond, then a single covalent bond is denoted. For example, when Alk^{a} and Y denote both a covalent bond and Alk^{b} is -CH₂-, then the moiety Alk^{a}-Y-Alk^{b} denotes -CH₂-. Similary, if Alk^{a}, Y and Alk^{b} each denote a covalent bond and L denotes H, then the moiety Alk^{a}-Y-Alk^{b}-L denotes -H.

The pharmaceutically acceptable salts are defined to comprise the therapeutically active non-toxic acid addition salts forms that the compounds according to Formula (I) are able to form. Said salts can be obtained by treating the base form of the compounds according to Formula (I) with appropriate acids, for example inorganic acids, for example hydrohalic acid, in particular hydrochloric acid, hydrobromic acid, sulfuric acid, nitric acid and phosphoric acid ; organic acids, for example acetic acid, hydroxyacetic acid, propanoic acid, lactic acid, pyruvic acid, oxalic acid, malonic acid, succinic acid, maleic acid, fumaric acid, malic acid, tartaric acid, citric acid, methanesulfonic acid, ethanesulfonic acid, benzenesulfonic acid, p-toluenesulfonic acid, cyclamic acid, salicylic acid, p-aminosalicylic acid and pamoic acid.

The compounds according to Formula (I) containing acidic protons may also be converted into their therapeutically active non-toxic metal or amine addition salts forms by treatment with appropriate organic and inorganic bases. Appropriate base salts forms comprise, for example, the ammonium salts, the alkaline and earth alkaline metal salts, in particular lithium, sodium, potassium, magnesium and calcium salts, salts with organic bases, e.g. the benzathine, *N*-methyl-D-glucamine, hybramine salts, and salts with amino acids, for example arginine and lysine.

Conversely, said salts forms can be converted into the free forms by treatment with an appropriate base or acid.

The term addition salt as used in the framework of this application also comprises the solvates that the compounds according to Formula (I) as well as the salts thereof, are able to form. Such solvates are, for example, hydrates and alcoholates.

The *N*-oxide forms of the compounds according to Formula (I) are meant to comprise those compounds of Formula (I) wherein one or several nitrogen atoms are oxidized to the so-called *N*-oxide, particularly those *N*-oxides wherein one or more tertiary nitrogens (e.g of the piperazinyl or piperidinyl radical) are *N*-oxidized. Such *N*-oxides can easily be obtained by a skilled person without any inventive skills and they are obvious alternatives for the compounds according to Formula (I) since these compounds are metabolites, which are formed by oxidation in the human body upon uptake. As is generally known, oxidation is normally the first step involved in drug metabolism (Textbook of Organic Medicinal and Pharmaceutical Chemistry, 1977, pages 70- 75). As is also generally known, the metabolite form of a compound can also be administered to a human instead of the compound per se, with much the same effects.

The compounds according to the invention possess at least 2 oxydizable nitrogens (tertiary amines moieties). It is therefore highly likely that *N*-oxides are to form in the human metabolism.

The compounds of Formula (I) may be converted to the corresponding *N*-oxide forms following art-known procedures for converting a trivalent nitrogen into its *N*-oxide form. Said *N*-oxidation reaction may generally be carried out by reacting the starting material of Formula (I) with an appropriate organic or inorganic peroxide. Appropriate inorganic peroxides comprise, for example, hydrogen peroxide, alkali metal or earth alkaline metal peroxides, e.g. sodium peroxide, potassium peroxide; appropriate organic peroxides may comprise peroxy acids such as, for example, benzenecarboperoxoic acid or halo substituted benzenecarboperoxoic acid, e.g. 3-chlorobenzenecarboperoxoic acid, peroxoalkanoic acids, e.g. peroxoacetic acid, alkylhydroperoxides, e.g. *tert*-butyl hydroperoxide. Suitable solvents are, for example, water, lower alkanols, e.g. ethanol and the like, hydrocarbons, e.g. toluene, ketones, e.g. 2-butanone, halogenated hydrocarbons, e.g. dichloromethane, and mixtures of such solvents.

The term "stereochemically isomeric forms" as used hereinbefore defines all the possible isomeric forms that the compounds of Formula (I) may possess. Unless otherwise mentioned or indicated, the chemical designation of compounds denotes the mixture of all possible stereochemically isomeric forms, said mixtures containing all diastereomers and enantiomers of the basic molecular structure. More in particular, stereogenic centers may have the R- or S-configuration; substituents on bivalent cyclic (partially) saturated radicals may have either the cis- or trans-configuration. Compounds encompassing double bonds can have an E or Z-stereochemistry at said double bond. Stereochemically isomeric forms of the compounds of Formula (I) are obviously intended to be embraced within the scope of this invention.

Following CAS nomenclature conventions, when two stereogenic centers of known absolute configuration are present in a molecule, an R or S descriptor is assigned (based on Cahn-Ingold-Prelog sequence rule) to the lowest-numbered chiral center, the reference center. R* and S* each indicate optically pure stereogenic centers with undetermined absolute configuration. If "α" and "β" are used : the position of the highest priority substituent on the asymmetric carbon atom in the ring system having the lowest ring number, is arbitrarily always in the "α" position of the mean plane determined by the ring system. The position of the highest priority substituent on the other asymmetric carbon atom in the ring system (hydrogen atom in compounds according to Formula (I)) relative to the position of the highest priority substituent on the reference atom is denominated "α", if it is on the same side of the mean plane determined by the ring system, or "β", if it is on the other side of the mean plane determined by the ring system.

Compounds according to Formula (I) and some of the intermediate compounds have at least two stereogenic centers in their structure.

The invention also comprises derivative compounds (usually called "pro-drugs") of the pharmacologically-active compounds according to the invention, which are degraded *in vivo* to yield the compounds according to the invention. Pro-drugs are usually (but not always) of lower potency at the target receptor than the compounds to which they are degraded. Pro-drugs are particularly useful when the desired compound has chemical or physical properties that make its administration difficult or inefficient. For example, the desired compound may be only poorly soluble, it may be poorly transported across the mucosal epithelium, or it may have an undesirably short plasma half-life. Further discussion on pro-drugs may be found in Stella, V. J. et al., "Prodrugs", Drug Delivery Systems, 1985, pp. 112-176, and Drugs, 1985, 29, pp. 455-473.

Pro-drugs forms of the pharmacologically-active compounds according to the invention will generally be compounds according to Formula (I), the pharmaceutically acceptable acid or base addition salts thereof, the stereochemically isomeric forms thereof and the *N*-oxide form thereof, having an acid group which is esterified or amidated. Included in such esterified acid groups are groups of the formula -COOR^{x}, where R^{x} is a C₁₋₆alkyl, phenyl, benzyl or one of the following groups : 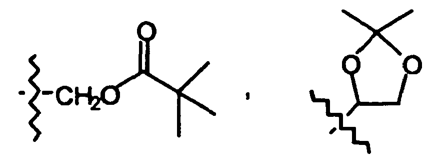 Amidated groups include groups of the formula - CONR^{y}R^{z}, wherein R^{y} is H, C₁₋₆alkyl, phenyl or benzyl and R^{z} is -OH, H, C₁₋₆alkyl, phenyl or benzyl. Compounds according to the invention having an amino group may be derivatised with a ketone or an aldehyde such as formaldehyde to form a Mannich base. This base will hydrolyze with first order kinetics in aqueous solution.

The compounds of Formula (I) as prepared in the processes described below may be synthesized in the form of racemic mixtures of enantiomers that can be separated from one another following art-known resolution procedures. The racemic compounds of Formula (I) may be converted into the corresponding diastereomeric salt forms by reaction with a suitable chiral acid. Said diastereomeric salt forms are subsequently separated, for example, by selective or fractional crystallization and the enantiomers are liberated therefrom by alkali. An alternative manner of separating the enantiomeric forms of the compounds of Formula (I) involves liquid chromatography using a chiral stationary phase. Said pure stereochemically isomeric forms may also be derived from the corresponding pure stereochemically isomeric forms of the appropriate starting materials, provided that the reaction occurs stereospecifically. Preferably if a specific stereoisomer is desired, said compound would be synthesized by stereospecific methods of preparation. These methods will advantageously employ enantiomerically pure starting materials.

### Pharmacology

The compounds of the present invention are potent inhibitors of neurokinin-mediated effects, in particular those mediated via the NK₁ and NK₃ receptor, and may therefore be described as neurokinin antagonists, especially as substance P antagonists, as may be indicated *in vitro* by the antagonism of substance P-induced relaxation of pig coronary arteries. The binding affinity of the present compounds for the human, guineapig and gerbil neurokinin receptors may also be determined *in vitro* in a receptor binding test using ³H-substance-P as radioligand. The subject compounds also show substance-P antagonistic activity *in vivo* as may be evidenced by, for instance, the antagonism of substance P-induced plasma extravasation in guinea-pigs, or the antagonism of drug-induced emesis in ferrets (Watson et al., Br. J. Pharmacol. 115:84-94 (1995)).

In view of their capability to antagonize the actions of tachykinins by blocking the neurokinin receptors, and in particular by blocking the NK₁ and NK₃ receptor, the compounds according to the invention are useful as a medicine, in particular in the prophylactic and therapeutic treatment of tachykinin-mediated conditions. In particular are compounds according to the invention are useful as orally active, centrally penetrating medicines in the prophylactic and therapeutic treatment of tachykinin-mediated conditions.

More in particular, it has been found that some compounds exhibit a combined NK₁/NK₃ antagonistic activity as can be seen from the Tables in the experimental section.

The invention therefore relates to a compound according to the general Formula (I), the pharmaceutically acceptable acid or base addition salts thereof, the stereochemically isomeric forms thereof, the *N*-oxide form thereof and prodrugs thereof, for use as a medicine.

The invention also relates to the use of a compound according to the general Formula (I), the pharmaceutically acceptable acid or base addition salts thereof, the stereochemically isomeric forms thereof, the *N*-oxide form thereof and prodrugs thereof for the manufacture of a medicament for treating, either prophylactic or therapeutic or both, neurokinin mediated conditions.

The compounds according to the invention are useful in the treatment of CNS disorders, in particular depression, anxiety disorders, stress-related disorders, sleep disorders, cognitive disorders, personality disorders, schizoaffective disorders, eating disorders, neurodegenerative diseases, addiction disorders, mood disorders, sexual dysfunction, pain and other CNS-related conditions ; inflammation ; allergic disorders ; emesis ; gastrointestinal disorders, in particular irritable bowel syndrome (IBS); skin disorders ; vasospastic diseases ; fibrosing and collagen diseases ; disorders related to immune enhancement or suppression and rheumatic diseases and body weight control.

In particular, the compounds according to the invention are useful in the treatment or prevention of depression including but not limited to major depressive disorders including bipolar depression ; unipolar depression ; single or recurrent major depressive episodes with or without psychotic features, catatonic features, melancholic features, atypical features or postpartum onset, and, in the case of recurrent episodes, with or without seasonal pattern. Other mood disorders encompassed within the term "major depressive disorder" include dysthymic disorder with early or late onset and with or without atypical features, bipolar I disorder, bipolar II disorder, cyclothymic disorder, recurrent brief depressive disorder, mixed affective disorder, neurotic depression, post traumatic stress disorder and social phobia ; dementia of the Alzheimer's type with early or late onset, with depressed mood ; vascular dementia with depressed mood ; substance-induced mood disorders such as mood disorders induced by alcohol, amphetamines, cocaine, hallucinogens, inhalants, opioids, phencyclidine, sedatives, hypnotics, anxiolytics and other substances ; schizoaffective disorder of the depressed type ; and adjustment disorder with depressed mood. Major depressive disorders may also result from a general medical condition including, but not limited to, myocardial infarction, diabetes, miscarriage or abortion, etc.

In particular, the compounds according to the invention are useful in the treatment or prevention of anxiety disorders, including but not limited to panic attack ; agoraphobia ; panic disorder without agoraphobia ; agoraphobia without history of panic disorder ; specific phobia ; social phobia ; obsessive-compulsive disorder ; post-traumatic stress disorder ; acute stress disorder ; generalized anxiety disorder ; anxiety disorder due to a general medical condition ; substance-induced anxiety disorder ; and anxiety disorder not otherwise specified.

In particular, the compounds according to the invention are useful in the treatment or prevention of stress-related disorders associated with depression and/or anxiety, including but not limited to acute stress reaction ; adjustment disorders, such as brief depressive reaction, prolonged depressive reaction, mixed anxiety and depressive reaction, adjustment disorder with predominant disturbance of other emotions, adjustment disorder with predominant disturbance of conduct, adjustment disorder with mixed disturbance of emotions and conduct and adjustment disorders with other specified predominant symptoms ; and other reactions to severe stress.

In particular, the compounds according to the invention are useful in the treatment or prevention of sleep disorders, including but not limited to dysomnia and/or parasomnias as primary sleep disorders; insomnia; sleep apnea ; narcolepsy ; circadian rhythms disorders ; sleep disorders related to another mental disorder ; sleep disorder due to a general medical condition; and substance-induced sleep disorder.

In particular, the compounds according to the invention are useful in the treatment or prevention of cognitive disorders, including but not limited to dementia ; amnesic disorders and cognitive disorders not otherwise specified, especially dementia caused by degenerative disorders, lesions, trauma, infections, vascular disorders, toxins, anoxia, vitamin deficiency or endocrinic disorders ; dementia of the Alzheimer's type, with early or late onset, with depressed mood ; AIDS-associated dementia or amnesic disorders caused by alcohol or other causes of thiamin deficiency, bilateral temporal lobe damage due to Herpes simplex encephalitis and other limbic encephalitis, neuronal loss secondary to anoxia / hypoglycemia / severe convulsions and surgery, degenerative disorders, vascular disorders or pathology around ventricle III. Furthermore, the compounds according to the invention are also useful as memory and/or cognition enhancers in healthy humans with no cognitive and/or memory deficit.

In particular, the compounds according to the invention are useful in the treatment or prevention of personality disorders, including but not limited to paranoid personality disorder ; schizoid personality disorder ; schizotypical personality disorder ; antisocial personality disorder ; borderline personality disorder ; histrionic personality disorder ; narcissistic personality disorder ; avoidant personality disorder ; dependent personality disorder ; obsessive-compulsive personality disorder and personality disorder not otherwise specified.

In particular, the compounds according to the invention are useful in the treatment or prevention of schizoaffective disorders resulting from various causes, including schizoaffective disorders of the manic type, of the depressive type, of mixed type ; paranoid, disorganized, catatonic, undifferentiated and residual schizophrenia; schizophreniform disorder ; schizoaffective disorder ; delusional disorder ; brief psychotic disorder ; shared psychotic disorder ; substance-induced psychotic disorder ; and psychotic disorder not otherwise specified.

In particular, the compounds according to the invention are also useful in the treatment or prevention of eating disorders, including anorexia nervosa ; atypical anorexia nervosa ; bulimia nervosa ; atypical bulimia nervosa ; overeating associated with other psychological disturbances ; vomiting associated with other psychological disturbances ; and non-specified eating disorders.

In particular, the compounds according to the invention are also useful in the treatment or prevention of neurodegenerative diseases, including but not limited to Alzheimer's disease ; Huntington's chorea ; Creutzfeld-Jacob disease ; Pick's disease ; demyelinating disorders, such as multiple sclerosis and ALS ; other neuropathies and neuralgia; multiple sclerosis ; amyotropical lateral sclerosis ; stroke and head trauma.

In particular, the compounds according to the invention are also useful in the treatment or prevention of addiction disorders including but not limited to substance dependence or abuse with or without physiological dependence, particularly where the substance is alcohol, amphetamines, amphetamine-like substances, caffeine, cocaine, hallucinogens, inhalants, nicotine, opioids (such as cannabis, heroin and morphine), phencyclidine, phencyclidine-like compounds, sedative-hypnotics, benzodiazepines and/or other substances, particularly useful for treating withdrawal from the above substances and alcohol withdrawal delirium.

In particular, the compounds according to the invention are also useful in the treatment or prevention of mood disorders induced particularly by alcohol, amphetamines, caffeine, cannabis, cocaine, hallucinogens, inhalants, nicotine, opioids, phencyclidine, sedatives, hypnotics, anxiolytics and other substances.

In particular, the compounds according to the invention are also useful in the treatment or prevention of sexual dysfunction, including but not limited to sexual desire disorders ; sexual arousal disorders ; orgasmic disorders ; sexual pain disorders ; sexual dysfunction due to a general medical condition ; substance-induced sexual dysfunction and sexual dysfunction not otherwise specified.

In particular, the compounds according to the invention are also useful in the treatment or prevention of pain, including but not limited to traumatic pain such as postoperative pain ; traumatic avulsion pain such as brachial plexus ; chronic pain such pancreatitis induced chronic pain or arthritic pain such as occurring in osteo- rheumatoid or psoriatic arthritis; neuropathic pain such as post-herpetic neuralgia, trigeminal neuralgia, segmental or intercostal neuralgia, fibromyalgia, causalgia, peripheral neuropathy, diabetic neuropathy, chemotherapy-induced neuropathy, AIDS related neuropathy, occipital neuralgia, geniculate neuralgia, glossopharyngeal neuralgia, reflex sympathetic dystrophy and phantom limb pain ; various forms of headache such as migraine, acute or chronic tension headache, temporomandibular pain, maxillary sinus pain and cluster headache ; odontalgia ; cancer pain ; visceral pain ; gastrointestinal pain ; nerve entrapment pain ; sport's injury pain ; dysmennorrhoea ; menstrual pain ; meningitis ; arachnoiditis ; musculoskeletal pain ; low back pain such as spinal stenosis, prolapsed disc, sciatica, angina, ankylosing spondyolitis ; gout ; bums ; scar pain ; itch ; and thalamic pain such as post stroke thalamic pain.

In particular, the compounds according to the invention are also useful in the treatment or prevention of the following other CNS-related conditions : akinesia, akinetic-rigid syndromes, dyskinesia and medication-induced parkinsonism, Gilles de la Tourette syndrome and its symptoms, tremor, chorea, myoclonus, tics and dystonia, attention-deficit / hyperactivity disorder (ADHD), Parkinson's disease, drug-induced Parkinsonism, post-encephalitic Parkinsonism, progressive supranuclear palsy, multiple system atrophy, corticobasal degeneration, parkinsonism-ALS dementia complex and basal ganglia calcification, behavioral disturbances and conduct disorders in dementia and the mentally retarded, including restlessness and agitation, extra-pyramidal movement disorders, Down's syndrome and Akathisia.

In particular, the compounds according to the invention are also useful in the treatment or prevention of inflammation, including but not limited to inflammatory conditions in asthma, influenza, chronic bronchitis and rheumatoid arthritis ; inflammatory conditions in the gastrointestinal tract such as, but not limited to Crohn's disease, ulcerative colitis, inflammatory bowel disease and non-steroidal antiinflammatory drug induced damage ; inflammatory conditions of the skin such as herpes and eczema ; inflammatory conditions of the bladder such as cystitis and urge incontinence ; and eye and dental inflammation and pancreatitis, in particular chronic and acute pancreatitis.

In particular, the compounds according to the invention are also useful in the treatment or prevention of allergic disorders, including but not limited to allergic disorders of the skin such as but not limited to urticaria ; and allergic disorders of the airways such as but not limited to rhinitis.

In particular, the compounds according to the invention are also useful in the treatment or prevention of emesis, i.e. nausea, retching and vomiting, including but not limited to acute emesis, delayed emesis and anticipatory emesis ; emesis induced by drugs such as cancer chemotherapeutic agents such as alkylating agents, for example cyclophosphamide, carmustine, lomustine and chlorambucil ; cytotoxic antibiotics, for example dactinomycin, doxorubicin, mitomycin-C and bleomycin ; anti-metabolites, for example cytarabine, methotrexate and 5-fluorouracil ; vinca alkaloids, for example etoposide, vinblastine and vincristine ; and other drugs such as cisplatin, dacarbazine, procarbazine and hydroxyurea ; and combinations thereof; radiation sickness ; radiation therapy, such as in the treatment of cancer; poisons ; toxins such as toxins caused by metabolic disorders or by infection, such as gastritis, or released during bacterial or viral gastrointestinal infection ; pregnancy ; vestibular disorders, such as motion sickness, vertigo, dizziness and Meniere's disease ; post-operative sickness; gastrointestinal obstruction; reduced gastrointestinal motility; visceral pain, such as myocardial infarction or peritonitis ; migraine ; increased intracranial pressure ; decreased intracranial pressure (such as altitude sickness); opioid analgesics, such as morphine; gastro-oesophageal reflux disease ; acid indigestion ; over-indulgence of food or drink ; acid stomach ; sour stomach ; waterbrash/regurgitation ; heartburn, such as episodic heartburn, nocturnal heartburn and meal induced heartburn ; and dyspepsia.

In particular, the compounds according to the invention are also useful in the treatment or prevention of gastrointestinal disorders, including but not limited to irritable bowel syndrome (IBS), skin disorders such as psoriasis, pruritis and sunburn ; vasospastic diseases such as angina, vascular headache and Reynaud's disease, cerebral ischaemia such as cerebral vasospasm following subarachnoid haemorrhage ; fibrosing and collagen diseases such as scleroderma and eosinophilic fascioliasis ; disorders related to immune enhancement or suppression such as systemic lupus erythematosus and rheumatic diseases such as fibrositis ; cough ; and body weight control, including obesity.

Most in particular, the compounds according to the invention are useful for the manufacture of a medicament for the treatment and/or prophylaxis of schizophrenia, emesis, anxiety and depression, irritable bowel syndrome (IBS), circadian rhythm disturbances, pre-eclampsia, nociception, pain, in particular visceral and neuropathic pain, pancreatitis, neurogenic inflammation, asthma, chronic obstructive pulmonary disease (COPD) and micturition disorders such as urinary incontinence.

The present invention also relates to a method for the treatment and/or prophylaxis of neurokinin-mediated diseases, in particular for the treatment and/or prophylaxis of schizophrenia, emesis, anxiety and depression, irritable bowel syndrome (IBS), circadian rhythm disturbances, pre-eclampsia, nociception, pain, in particular visceral and neuropathic pain, pancreatitis, neurogenic inflammation, asthma, chronic obstructive pulmonary disease (COPD) and micturition disorders such as urinary incontinence, comprising administering to a human in need of such administration an effective amount of a compound according to the invention, in particular according to Formula (I), the pharmaceutically acceptable acid or base addition salts thereof, the stereochemically isomeric forms thereof, the *N*-oxide form thereof, as well as the prodrugs thereof.

The invention also relates to a pharmaceutical composition comprising a pharmaceutically acceptable carrier and, as active ingredient, a therapeutically effective amount of a compound according to the invention, in particular a compound according to Formula (I), the pharmaceutically acceptable acid or base addition salts thereof, the stereochemically isomeric forms thereof, the N-oxide form thereof and a prodrug thereof

The compounds according to the invention, in particular the compounds according to Formula (I), the pharmaceutically acceptable acid or base addition salts thereof, the stereochemically isomeric forms thereof, the *N*-oxide form thereof and the prodrugs thereof, or any subgroup or combination thereof may be formulated into various pharmaceutical forms for administration purposes. As appropriate compositions there may be cited all compositions usually employed for systemically administering drugs. To prepare the pharmaceutical compositions of this invention, an effective amount of the particular compound, optionally in addition salt form, as the active ingredient is combined in intimate admixture with a pharmaceutically acceptable carrier, which carrier may take a wide variety of forms depending on the form of preparation desired for administration. These pharmaceutical compositions are desirable in unitary dosage form suitable, in particular, for administration orally, rectally, percutaneously, by parenteral injection or by inhalation. For example, in preparing the compositions in oral dosage form, any of the usual pharmaceutical media may be employed such as, for example, water, glycols, oils, alcohols and the like in the case of oral liquid preparations such as suspensions, syrups, elixirs, emulsions and solutions; or solid carriers such as starches, sugars, kaolin, diluents, lubricants, binders, disintegrating agents and the like in the case of powders, pills, capsules and tablets. Because of their ease in administration, tablets and capsules represent the most advantageous oral dosage unit forms in which case solid pharmaceutical carriers are obviously employed. For parenteral compositions, the carrier will usually comprise sterile water, at least in large part, though other ingredients, for example, to aid solubility, may be included. Injectable solutions, for example, may be prepared in which the carrier comprises saline solution, glucose solution or a mixture of saline and glucose solution. Injectable suspensions may also be prepared in which case appropriate liquid carriers, suspending agents and the like may be employed. Also included are solid form preparations that are intended to be converted, shortly before use, to liquid form preparations. In the compositions suitable for percutaneous administration, the carrier optionally comprises a penetration enhancing agent and/or a suitable wetting agent, optionally combined with suitable additives of any nature in minor proportions, which additives do not introduce a significant deleterious effect on the skin. Said additives may facilitate the administration to the skin and/or may be helpful for preparing the desired compositions. These compositions may be administered in various ways, e.g., as a transdermal patch, as a spot-on, as an ointment.

It is especially advantageous to formulate the aforementioned pharmaceutical compositions in unit dosage form for ease of administration and uniformity of dosage. Unit dosage form as used herein refers to physically discrete units suitable as unitary dosages, each unit containing a predetermined quantity of active ingredient calculated to produce the desired therapeutic effect in association with the required pharmaceutical carrier. Examples of such unit dosage forms are tablets (including scored or coated tablets), capsules, pills, powder packets, wafers, suppositories, injectable solutions or suspensions and the like, and segregated multiples thereof.

Since the compounds according to the invention are potent orally administrable NK₁ and NK₁/NK₃ antagonists, pharmaceutical compositions comprising said compounds for administration orally are especially advantageous.

### Synthesis

The compounds according to the invention can generally be prepared by a succession of steps, each of which is known to the skilled person.

The compounds of Formula (I) are conveniently prepared by reductively N-alkylating an intermediate of Formula (II) wherein R¹, R², R⁴, X, Q, m, n, p and Z are defined as in Formula (I), with a *N*-substituted piperidinon of Formula (III) wherein R¹, Alk, Y, L, j, k and q are defined as in Formula (I). Said reductive *N*-alkylation may be performed in a reaction-inert solvent such as, for example, dichloromethane, ethanol or toluene or a mixture thereof, and in the presence of an appropriate reducing agent such as, for example, a borohydride, e.g. sodium borohydride, sodium cyanoborohydride or triacetoxy borohydride. In case a borohydride is used as a reducing agent, it may be convenient to use a complex-forming agent such as, for example, titanium(TV)-isopropylate as described in J. Org. Chem, 1990, 55, 2552-2554. Using said complex-forming agent may also result in an improved *cis*/*trans* ratio in favor of the *trans* isomer. It may also be convenient to use hydrogen as a reducing agent in combination with a suitable catalyst such as, for example, palladium-on-charcoal or platinum-on-charcoal. In case hydrogen is used as reducing agent, it may be advantageous to add a dehydrating agent to the reaction mixture such as, for example, aluminium *tert*-butoxide. In order to prevent the undesired further hydrogenation of certain functional groups in the reactants and the reaction products, it may also be advantageous to add an appropriate catalyst-poison to the reaction mixture, e.g., thiophene or quinoline-sulphur. Stirring and optionally elevated temperatures and/or pressure may enhance the rate of the reaction.

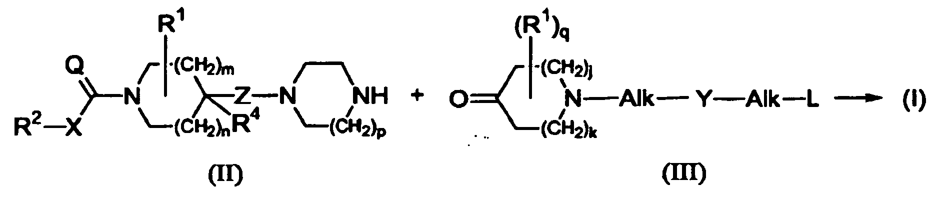

In this and the following preparations, the reaction products may be isolated from the reaction medium and, if necessary, further purified according to methodologies generally known in the art such as, for example, extraction, crystallization, trituration and chromatography.

Especially advantage is the preparation of a compound according to the invention according to the previous reaction scheme in which the Alk-Y-Alk-L-moiety is benzyl, thus giving rise to a compound according to Formula (I) in which the Alk-Y-Alk-L-moiety is benzyl. Said compound is pharmacological active and can be converted into a compound according to the invention in which the Alk-Y-Alk-L-moiety is hydrogen by reductive hydrogenation using e.g. hydrogen as a reducing agent in combination with a suitable catalyst such as, for example, palladium-on-charcoal or platinum-on-charcoal. The resulting compound according to the invention can then be converted into other compounds according to the invention by art-known transformations, e.g. acylation and alkylation.

In particular, the compounds of Formula (I^{a}) can be prepared by reacting a final compound of Formula (I') wherein R¹, R², R⁴, X, Q, Z, m, n, p and q are defined as in Formula (I), with an acyl compound of Formula (V) wherein Alk and L are defined as in Formula (I) and W¹ is an appropriate leaving group such as, for example, a halo, e.g. chloro or bromo, or a sulfonyloxy leaving group, e.g. methanesulfonyloxy or benzenesulfonyloxy. The reaction can be performed in a reaction-inert solvent such as, for example, a chlorinated hydrocarbon, e.g. dichloromethane, an alcohol, e.g. ethanol, or a ketone, e.g. methyl isobutylketone, and in the presence of a suitable base such as, for example, sodium carbonate, sodium hydrogen carbonate or triethylamine. Stirring may enhance the rate of the reaction. The reaction may conveniently be carried out at a temperature ranging between room temperature and reflux temperature.

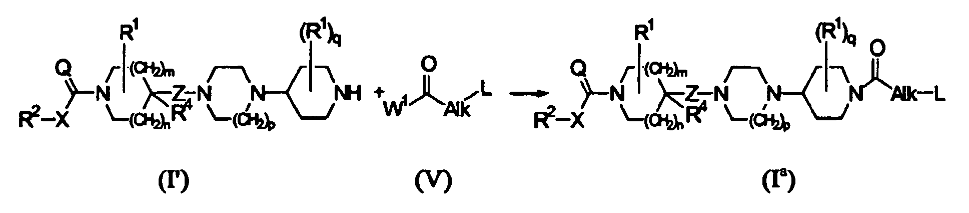

Alternatively, the compounds of Formula (I^{a}) can also be prepared by reacting a final compound of Formula (I') wherein R¹, R², R⁴, X, Q, Z, m, n, p and q are defined as in Formula (I) with a carboxylic acid of Formula (VI) wherein Alk and L are defined as in Formula (I)(base-catalyzed nucleophilic addition reaction). The reaction can be performed in a reaction-inert solvent such as, for example, a chlorinated hydrocarbon, e.g. dichloromethane, an alcohol, e.g. ethanol, or a ketone, e.g. methyl isobutylketone, and in the presence of a suitable base such as, for example, sodium carbonate, sodium hydrogen carbonate or triethylamine. Stirring may enhance the rate of the reaction. The reaction may conveniently be carried at a temperature ranging between room temperature and reflux temperature. 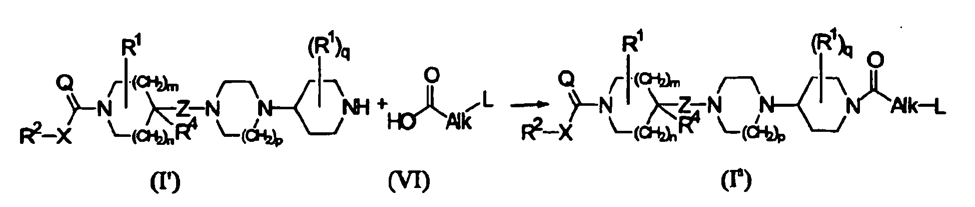 The above reaction may also be earned out under equivalent conditions with the carboxylic ester of the carboxylic acid of Formula (VI).

In particular, the compounds of Formula (I^{b}) can be prepared by reacting a final compound of Formula (T) wherein R¹, R², R⁴, X, Q, Z, m, n, p and q are defined as in Formula (I) with a keto-compound of Formula (VII) wherein Alk and L are defined as in Formula (I) and wherein W² is an appropriate leaving group such as, for example, a halogen, e.g. chloro or bromo, or a sulfonyloxy leaving group, e.g. methanesulfonyloxy or benzenesulfonyloxy. The reaction can be performed in a reaction-inert solvent such as, for example, a chlorinated hydrocarbon, e.g. dichloromethane, an alcohol, e.g. ethanol, or a ketone, e.g. methyl isobutylketone, and in the presence of a suitable base such as, for example, sodium carbonate, sodium hydrogen carbonate or triethylamine. Stirring may enhance the rate of the reaction. The reaction may conveniently be carried at a temperature ranging between room temperature and reflux temperature.

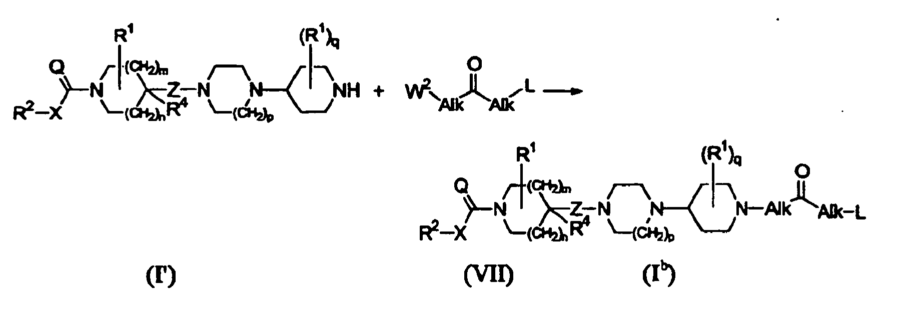

The compounds of Formula (I^{c}) can be prepared by reductive amination/alkylation of a final compound of Formula (I') wherein R¹, R², R⁴, X, Q, Z, m, n, p and q are defined as in Formula (I) with a compound of Formula (VIII) wherein Alk and L are defined as in Formula (I) and W³ is an appropriate leaving group such as, for example, a halogen, e.g. chloro or bromo, or a sulfonyloxy leaving group, e.g. methanesulfonyloxy or benzenesulfonyloxy. The reaction can be performed in a reaction-inert solvent such as, for example, a chlorinated hydrocarbon, e.g. dichloromethane, an alcohol, e.g. ethanol, or a ketone, e.g. methyl isobutylketone, and in the presence of a suitable base such as, for example, sodium carbonate, sodium hydrogen carbonate or triethylamine. Stirring may enhance the rate of the reaction. The reaction may conveniently be carried at a temperature ranging between room temperature and reflux temperature.

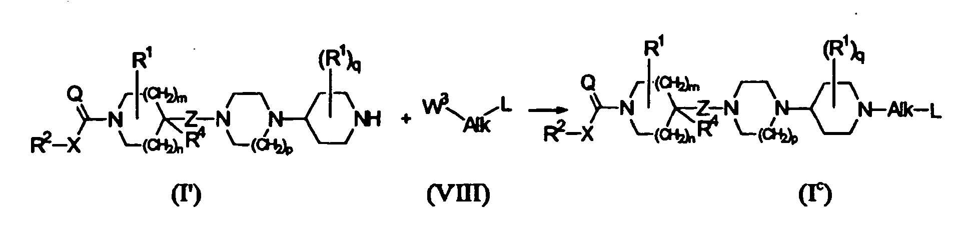

The starting materials and some of the intermediates are known compounds and are commercially available or may be prepared according to conventional reaction procedures generally known in the art. The preparation of intermediates is i.a. described in the experimental section, in WO 97/16440-A1, published May 9, 1997 by Janssen Pharmaceutica N.V, which is disclosed herein by reference as well as in other publications mentioned in WO 97/16440-A1, such as , e.g. EP-0,532,456-A.

Compounds according to the invention may be converted into each other following art-known transformation reactions, such as illustrated further.

More specifically, compounds of Formula (XIII), wherein A is an aryl or heteroaryl, K may be any moiety, preferably a moiety K¹ as defined below, Het is an unsaturated heteroaryl and r is an integer ranging from 1 to a number equal to the number of available carbon atoms in the aryl or heteroaryl-moiety A, e.g. 5 in phenyl and 4 in pyrrolyl, may be obtained by a novel type of Heck-reaction wherein a compound of Formula (XI), wherein K, A and r are as defined in Formula (XIII) and Hal is a halogen, thus comprising an active or non-active halo-substituted aryl or halo-substituted heteroaryl, more preferably a mono or polysubstituted bromo- and/or iodoaryl or -heteroaryl moiety is reacted with an unsaturated heteroaryl according to Formula (XII) in the presence of catalytic amounts of Pd(OAc)₂ and 1,3-bis diphenylphosphinopropane, in the presence of a suitable base, preferably Cs₂CO₃ or K(AcO), in a reaction-inert polar solvent such as, preferably NMP, DMA, DMF or the like and at an elevated reaction temperature, preferably at 140-150 °C for a certain period of time, preferably about 6-20 hours, more preferably 12-18 hours.

Preferably, r is 1. Het may be a unsaturated monocyclic or bicyclic heteroaryl moiety, such as for instance imidazo[1,2-a]pyridinyl, pyrrolyl, thienyl, thiazolyl, imidazolyl, oxazolyl, furanyl, thienyl, benzimidazolyl, benzoxazolyl, benzthiazolyl, benzofuranyl, benzothienyl or indolyl or such as any of the unsaturated radicals in the groups Het¹ and Het² as defined in Formula (I), optionally substituted with one or more radicals selected from the group of Ar¹, Ar¹alkyl, halo, hydroxy, alkyl, piperidinyl, pyrrolyl, thienyl, oxo, alkyloxy, alkyloxyalkyl and alkyloxycarbonyl. Preferably, A is phenyl or pyridinyl.

Said reaction has an improved yield over the process according to the prior art (Yutaka Aoyagi et al., Heterocycles, 1992, 33, 257 and Sommai Pivsa-Art, Bull.Chem.Soc. Jpn., 1998, 71, 467).

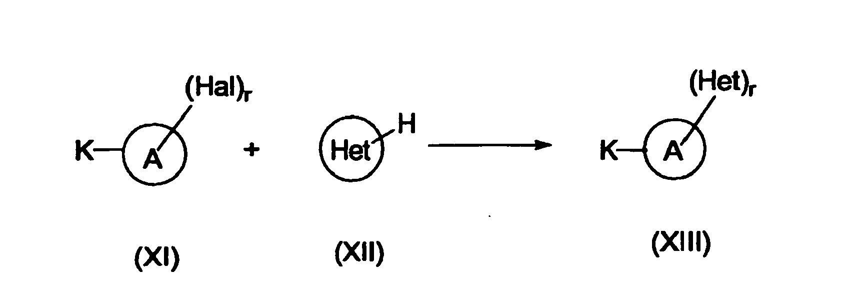

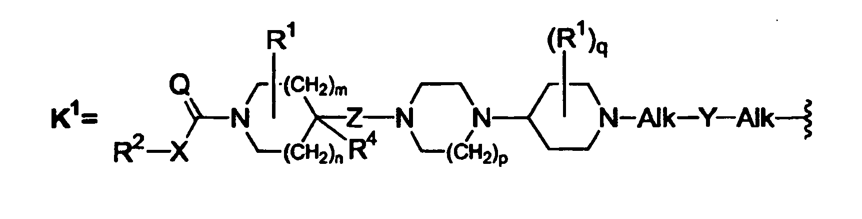

Also, compounds according to the invention may be converted into an acid addition salt by treatment with an acid, or into a base addition salt by treatment with a base, or conversely, the acid addition salt form may be converted into the free base by treatment with alkali, or the base addition salt may be converted into the free acid by treatment with an acid.

The following examples are intended to illustrate and not to limit the scope of the present invention.

### Experimental Part

Hereinafter "RT" means room temperature, "CDI" means 1,1'-carbonyldiimidazole, "DIPE" means diisopropylether, "MIK" means methyl isobutyl keton, "BINAP" means [1,1'-binaphthalene]-2,2'-diylbis[diphenylphosphine], "NMP" means 1-methyl-2-pyrrolidinone, "Pd₂(dba)₃" means tris(dibenzylideneacetone)dipalladium, "DMF" means *N,N*-dimethylformamid, "DMAP" means *N,N*-dimethyl-4-pyridinamine, "EDCI" means 1-(3-dimethylaminopropyl)-3-ethylcarbodiimide .HCl and "HOBT" means hydroxybenzotriazole.

### Preparation of the intermediate compounds

### Example A1

### a. Preparation of intermediate compound 1

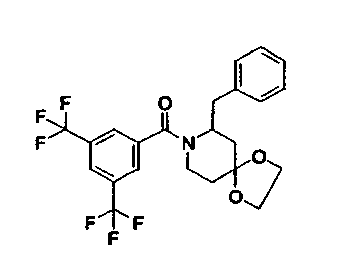

Et₃N (0.55 mol) was added to a stirring mixture of 7-(phenylmethyl)-1,4-dioxa-8-azaspiro[4.5]decane (prepared according to teachings in WO97/24350 of which the content is herein included by reference) (0.5 mol) in toluene (1500 ml). 3,5-Bis(trifluoromethyl)benzoyl chloride (0.5 mol) was added over a 1-hour period (exothermic reaction). The mixture was stirred at room temperature for 2 hours, then allowed to stand for the weekend and washed three times with water (500 ml, 2 x 250 ml). The organic layer was separated, dried, filtered and the solvent was evaporated, yielding 245 g of fraction 1 (100 %). Part of this fraction was crystallized from petroleum ether. The precipitate was filtered off and dried, yielding 1.06 g of intermediate compound 1.

### b. Preparation of intermediate compound 2 and 3

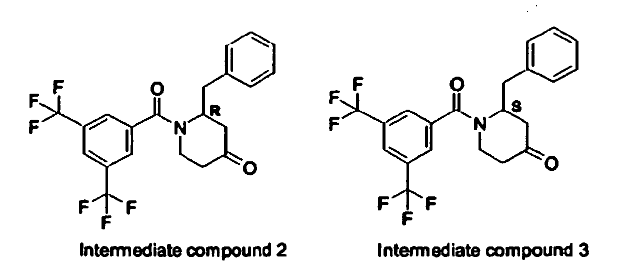

HCl cp (300 ml) was added to a mixture of intermediate compound 1 (prepared according to A1 .a) (0.5 mol) in ethanol (300 ml) and H₂O (300 ml). The reaction mixture was stirred at 60 °C for 20 hours. The precipitate was filtered off, ground, stirred in H₂O, filtered off, washed with petroleum ether and dried, yielding 192 g of fraction 1 (89.4 %). Part (75 g) of this fraction was separated into its optical isomers by chiral column chromatography over Chiralcel AS (20 µm, 100 Å; eluent: hexane/2-propanol 70/30). Two fraction groups were collected and their solvent was evaporated. Yield: 36.9 g of intermediate compound 2 and 37.5 g of intermediate compound 3.

### c. Preparation of intermediate compound 4

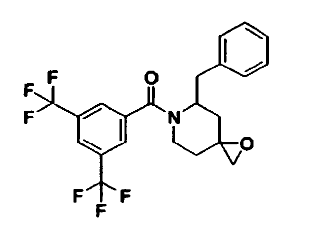

NaH, 60 % (0.04 mol) was washed 3 times with hexane, then DMSO (30 ml) was added and the mixture was stirred for 1 hour under N₂ and at 60 °C. The mixture was cooled to room temperature and THF (40 ml) was added. The resulting mixture was cooled to 0 °C and a solution of trimethylsulfoxonium iodide (0.032 mol) in DMSO (40 ml) was added dropwise. After stirring for 30 min. at 0°C, a solution of intermediate compound 2 (prepared according to A1.b) (0.02 mol) in DMSO (20 ml) was added dropwise and the reaction mixture was stirred for 3 hours at room temperature. The mixture was poured out into ice, washed with a NH₄Cl soln. and the layers were separated. The organic layer was dried (MgSO₄), filtered off and the solvent was evaporated. Yield: 7.3 g of intermediate compound 4 (83 %).

### Example A2

### a. Preparation of intermediate compound 5

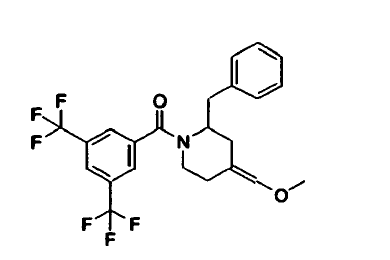

Reaction under N₂ atmosphere. A mixture of (methoxymethyl)triphenylphosphonium chloride (0.0055 mol) and *N*-(1-methylethyl)-2-propanamine (0.0083 mol) in THF, p.a., dry (20 ml) was stirred at -70 °C. 2.3 M BuLi/hexane (0.0055 mol) was added dropwise. More 2.3 M BuLi/hexane (2.2 ml) was added. The mixture was allowed to warm to room temperature. The reaction mixture was stirred for 30 min at 20 °C. The mixture was re-cooled to -25 °C. A solution of 1-[3.5-bis(trifluoromethyl)benzoyl]-2-(phenylmethyl)-4-piperidinone (prepared according to teachings in WO97/24350 of which the content is herein included by reference) (0.005 mol) in some THF, p.a,dry was added dropwise and the reaction mixture was allowed to warm to room temperature. The reaction mixture was stirred overnight at room temperature, then decomposed with water. The organic solvent was evaporated and the aqueous concentrate was extracted with CH₂Cl₂. The separated organic layer was dried, filtered and the solvent evaporated. The residue was purified over silica gel on a glass filter (eluent CH₂Cl₂). The desired fractions were collected and the solvent was evaporated. Yield: 1.30 g of intermediate compound 5 (57 %).

### b. Preparation of intermediate compound 6

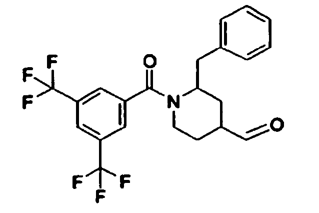

A mixture of intermediate compound 5 (prepared according to A2.a) (0.0028 mol) in 1.6 N HCl (6 ml) and THF (6 ml) was stirred for one hour at 40 °C. The THF was evaporated and the aqueous concentrate was extracted with CH₂Cl₂. The separated organic layer was washed with an aqueous Na₂CO₃ solution, dried, filtered and the solvent evaporated. Yield: 1.24 g of intermediate compound 6 (100 %).

### c. Preparation of intermediate compound 7

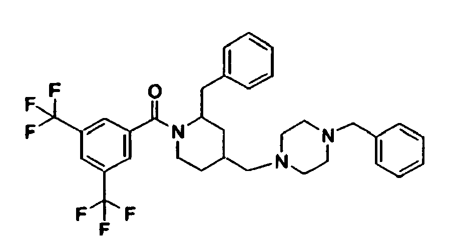

A mixture of intermediate compound 6 (prepared according to A2.b) (0.0028 mol) and 1-(phenylmethyl)piperazine (0.0028 mol) in THF (5 ml) and methanol (100 ml) was hydrogenated with Pd/C, 10 % (0.5 g) as a catalyst in the presence of thiophene solution (0.5 ml). After uptake of H₂ (1 eq.), the catalyst was filtered off and the filtrate was evaporated. The residue was treated with (E)-2-butenedioic acid in methanol. The salt was filtered off and re-converted into the free base with 50 % NaOH, then extracted with CH₂Cl₂. The separated organic layer was dried, filtered and the solvent evaporated. The residue solidified spontaneously and was dried. Yield: 0.56 g of intermediate compound 7 (33 %).

### d. Preparation of intermediate compound 8

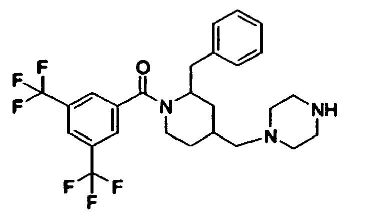

A mixture of intermediate compound 7 (prepared according to A2.c) (0.133 mol) in methanol (500 ml) was hydrogenated over the weekend with Pd/C, 10 % (5 g) as a catalyst. After uptake of H₂ (1eq.), the catalyst was filtered off and the filtrate was evaporated Yield: 67.5 g of intermediate compound 8.

### Example A3

### Preparation of intermediate compound 9

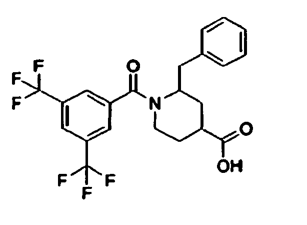

A mixture of intermediate compound 6 (prepared according to A2.b) (0.0081 mol) in CH₃CN (p.a) (20 ml) was stirred on an ice bath, giving mixture (I). A mixture of NaOCl₂ (0.00975 mol) dissolved in H₂O (20 ml) was added dropwise at <10 °C to mixture (I). The reaction mixture was stirred for 2 hours at <10°C and was stirred overnight at room temperature. NaOH (25 ml, 10 %) was added dropwise to the mixture at room temperature and the reaction mixture was washed with DIPE (2 times). The aqueous layer was acidified with HCl (10 %) at <10 °C, then extracted with CH₂Cl₂. The organic layer was dried (MgSO₄) and filtered off. The solvent was evaporated and the residue was crystallised from DIPE. Yield: 3.0 g of intermediate compound 9 (80 %).

### Example A4

### a. Preparation of intermediate compound 10

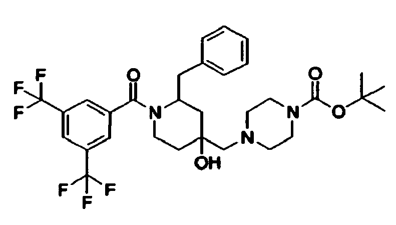

A mixture of intermediate compound 4 (prepared according to A1.c) (0.0165 mol) and 1,1-dimethylethyl-1-piperazinecarboxylic acid ester (0.0165 mol) in ethanol p.a. (100 ml) was stirred and refluxed for 14 hours. The solvent was evaporated and the residue was purified over silica gel on a glass filter (eluent: CH₂Cl₂/CH₃OH 97.5/2.5). The product fractions were collected and the solvent was evaporated. Yield: 8.3 g of intermediate compound 10 (80 %).

### b. Preparation of intermediate compound 11

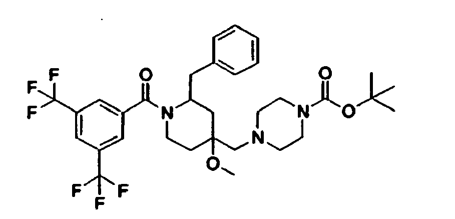

A mixture of intermediate compound 10 (prepared according to A4.a) (8.3 g; 0.0132 mol) in DMF p.a. (250 ml) was stirred at room temperature under N₂. NaH 60 % (0.64 g; 0.016 mol) was added and the reaction mixture was stirred for 1 hour at room temperature. A mixture of CH₃I (2.25 g; 0.0158 mol) dissolved in DMF was added dropwise. The reaction mixture was stirred for 18 hours at room temperature. Extra NaH 60 % (0.64 g) was added and the mixture was stirred for 1 hour at room temperature. Extra CH₃I (2.25 g) was added and the mixture was stirred for 18 hours at room temperature. Then the mixture was poured into H₂O and extracted with DIPE. The organic layer was dried with MgSO₄, filtered and evaporated. Yield: 6.4 g of intermediate compound 11 (75 %).

### c. Preparation of intermediate compound 12

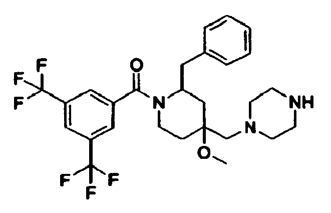

A mixture of intermediate compound 11 (prepared according to A4.b) (6.0 g; 0.0093 mol), HCl/iPrOH (25 ml) and methanol (100 ml) was stirred at 50 °C for 1 hour. The solvent was evaporated and the residue was poured out into H₂O. K₂CO₃ 10 % (3 g) was added. The filtrate was extracted with CH₂Cl₂. The organic layer was washed with H₂O, dried (MgSO₄), filtered, and the solvent was evaporated. Yield: 5.1 g of intermediate compound 12 (100 %).

### Example A5

### a) Preparation of intermediate compound 13

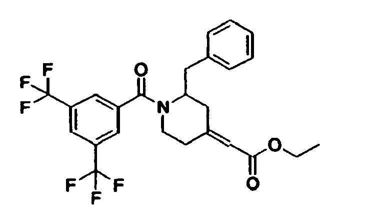

A mixture of NaH, 60 % (0.036 mol) in THF, p.a., dried on molecular sieves (200 ml) was stirred at room temperature and a solution of (diethoxyphosphinyl)acetic acid ethyl ester (0.036 mol) in THF, p.a., dried on molecular sieves (q.s.) was added dropwise, then the mixture was stirred for 30 min. at room temperature and a solution of intermediate compound 2 (prepared according to A1.b) (0.03 mol) in THF, p.a., dried on molecular sieves (q.s.) was added dropwise. The reaction mixture was stirred for 90 min. at room temperature. NH₄Cl (15 g) and H₂O (200 ml) were added. The organic solvent was evaporated and the aqueous concentrate was extracted with CH₂Cl₂. The organic layer was washed with H₂O, dried (MgSO₄), filtered off and the solvent was evaporated. The residue was purified over silica gel on a glass filter (eluent gradient: CH₂Cl₂/CH₃OH from 100/0 to 98/2). The product fractions were collected and the solvent was evaporated. Yield: 14.3 g of intermediate compound 13 (95 %).

### b) Preparation of intermediate compound 14

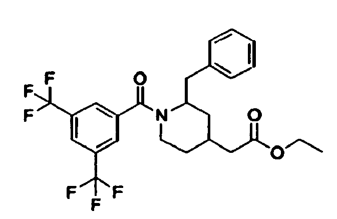

A mixture of intermediate compound 13 (prepared according to A5.a) (0.0126 mol) in ethanol (100 ml) was hydrogenated at 20°C for 18 hours with Pd/C 10 % (1 g) as a catalyst. After uptake of H₂ (1 eq.), the catalyst was filtered off and the filtrate was evaporated. Yield: 7.1 g of intermediate compound 14 (100 %).

### c) Preparation of intermediate compound 15

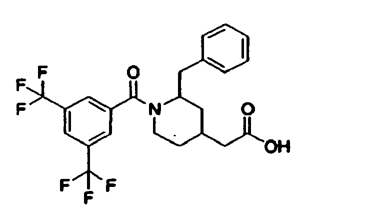

A mixture of intermediate compound 14 (prepared according to A5.b) (0.014 mol) in NaOH (1N) (75 ml), methanol (70 ml) and H₂O (70 ml) was stirred at room temperature for 24 hours. The organic solvent was evaporated, HCl (100 ml, 1N) was added and the reaction mixture was extracted (2 times) with CH₂Cl₂. The organic layer was washed with H₂O, dried (MgSO₄), filtered off and the solvent was evaporated. Yield: 5.5 g of intermediate compound 15 (83 %).

### Example A6

### Preparation of intermediate compound 16

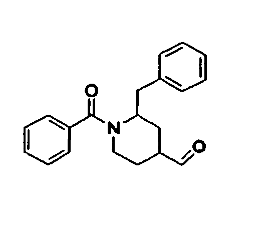

A mixture of intermediate compound 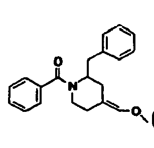 (2R) (prepared according to A2.a) (0.0133 mol) in 37 % HCl (10 ml), H₂O (30 ml) and THF (40 ml) was stirred for 2 hours at 50 °C, then the organic solvent was evaporated and the aqueous concentrate was extracted with CH₂Cl₂. The organic layer was washed with a NaHCO₃ soln. and with H₂O, dried (MgSO₄), filtered off and the solvent was evaporated. The residue was purified by column chromatography over silica gel (eluent gradient: CH₂Cl₂/CH₃OH from 100/0 to 98/2). The product fractions were collected and the solvent was evaporated. Yield: 2.7 g of intermediate compound 16 (66 %).

### Example A7

### Preparation of intermediate compound 17

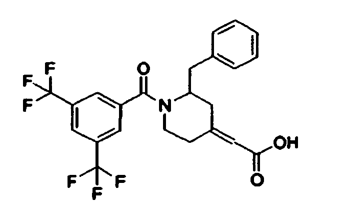

A mixture of intermediate compound 13 (prepared according to A5.a) (0.0143 mol) in NaOH (IN) (75 ml), methanol (170 ml) and H₂O (100 ml) was stirred at room temperature for 48 hours. The solvent was evaporated until water and the residue was washed with DIPE. HCl (100 ml, IN) was added and the reaction mixture was extracted with CH₂Cl₂. The organic layer was dried (MgSO₄), filtered off and the solvent was evaporated. The residue was purified by column chromatography over silica gel (eluent gradient: CH₂Cl₂/CH₃OH from 100/0 to 90/10). The product fractions were collected and the solvent was evaporated. Yield: 5.9 g of intermediate compound 17 (88 %).

### Preparation of the final compounds

### Example B1

### a) Preparation of final compound 1

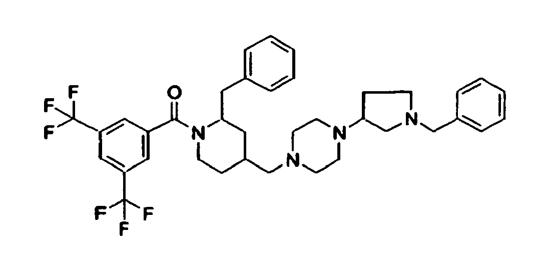

A mixture of intermediate compound 8 (prepared according to A2.d) (0.04 mol) and 1-(phenylmethyl)-3-pyrrolidinone (0.04 mol) in methanol (250 ml) was hydrogenated at 50 °C for 18 hours with Pd/C 10 % (3 g) as a catalyst in the presence of thiophene solution (2 ml). After uptake of H₂ (1eq.), the catalyst was filtered off and the filtrate was evaporated. The residue was taken up in H₂O/CH₂Cl₂ and K₂CO₃ (5 g). The layers were separated. The aqueous layer was extracted with CH₂Cl₂ and the organic layer was dried (MgSO₄), filtered off and the solvent was evaporated. The residue was purified by column chromatography over silica gel (eluent gradient: CH₂Cl₂/CH₃OH from 95/5 to 90/10). The product fractions were collected and the solvent was evaporated. Yield: 22.0 g of final compound 1 (82 %).

### b) Preparation of final compound 2

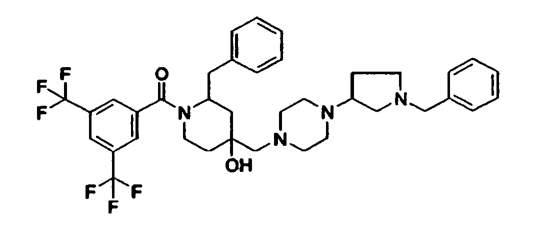

A mixture of intermediate compound 4 (prepared according to A1.c) (0.0165 mol) and 1-[1-(phenylmethyl)-3-pyrrolidinyl]piperazine (prepared according to teachings in WO96/20173, of which the content is herein included by reference) (0.0165 mol) in ethanol p.a. (100 ml) was stirred and refluxed overnight The solvent was evaporated and the residue was purified over silica gel on a glass filter (eluent gradient: CH₂Cl₂/CH₃OH from 95/5 to 90/10). The product fractions were collected and the solvent was evaporated. Yield: 7.6 g of final compound 2 (67 %).

### c) Preparation of final compound 3

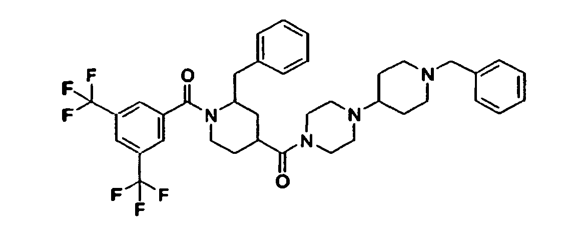

A mixture of intermediate compound 9 (prepared according to A3) (0.02 mol), *N,N-*dimethyl-4-pyridinamine (0.028 mol) and Et₃N (0.028 mol) in CH₂Cl₂, p.a. (300 ml) was stirred at room temperature, then EDCI (0.028 mol) was added portionwise and the reaction mixture was stirred for 5 hours at room temperature. A solution of 1-[1-(phenylmethyl)-4-piperidin-yl]Piperazine (prepared according to teachings in WO96/20173, of which the content is herein included by reference) (0.02 mol) in CH₂Cl₂ was added dropwise and the mixture was stirred over the weekend at room temperature. NaOH (150 ml, 1 N) was added and the reaction mixture was stirred for 15 min. at room temperature. The layers were separated and the aqueous layer was extracted with CH₂Cl₂. The organic layer was washed with H₂O, dried (MgSO₄), filtered off and the solvent was evaporated. Yield: 3.2 g of final compound 3 (23 %).

### d) Preparation of final compound 4

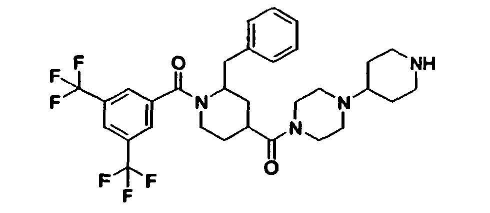

A mixture of final compound 3 (0.0041 mol) in H₂ (50 ml) was hydrogenated at room temperature for 72 hours with Pd/C 10 % (0.5 g) as a catalyst. After uptake of H₂ (1 eq.), the catalyst was filtered off and the filtrate was evaporated. Yield: 2.4 g of final compound 4 (96 %).

### Example B2

### a) Preparation of final compound 5

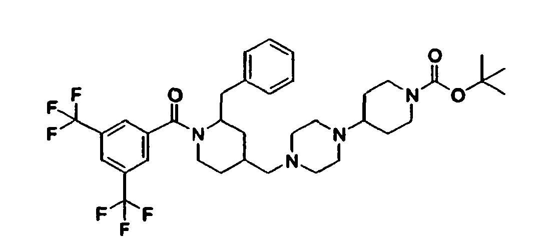

A mixture of intermediate compound 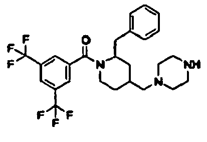 (trans) (prepared according to A2.d) (0.02 mol) and 1,1-dimethylethyl 4-oxo-1-piperidine-carboxylic acid ester (0.02 mol) in methanol (250 ml) was hydrogenated at 50°C with Pd/C 10 % (2 g) as a catalyst in the presence of thiophene solution (1 ml). After uptake of H₂ (1 eq.), the catalyst was filtered off and the solvent was evaporated. The residue was purified over silica gel on a glass filter (eluent gradient : CH₂Cl₂/MeOH from 97/3 to 94/6). The desired fractions were collected and the solvent was evaporated. Yield: 10.4 g of final compound 5 (75 %).

### b) Preparation of final compound 6

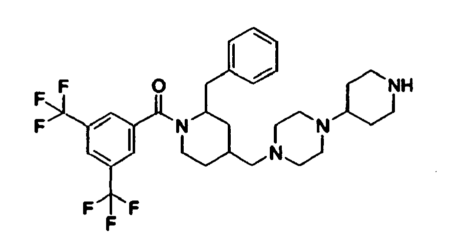

Final compound 5 (prepared according to B2.a) (0.0139 mol) in HCl/2-propanol (15 ml) and 2-propanol (60 ml) was stirred at 50 °C for 3 hours. The solvent was evaporated. Yield : 9.3 g of final compound 6 (95 %).

### Example B3

### a) Preparation of final compound 7

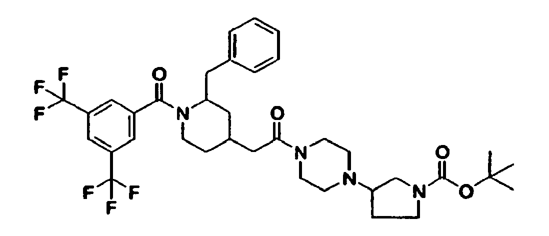

A mixture of intermediate compound 15 (prepared according to A5.c) (0.006 mol), DMAP (0.0077 mol) and Et₃N (0.0077 mol) in CH₂Cl₂ (100 ml) was stirred at room temperature. EDCI (0.0077 mol) was added and the mixture was stirred for 2 hours at room temperature, giving mixture (I). A mixture of 1,1-dimethylethyl 3-( 1-piperazinyl)-1-pyrrolidinecarboxylic acid ester ((prepared according to the teachings in WO2004/056799 of which the content is included herein) (0.006 mol) dissolved in CH₂Cl₂ was added dropwise to mixture (I) and the reaction mixture was stirred overnight at room temperature. H₂O (100 ml) and NaOH (50 ml, 1 N) were added and the mixture was stirred for 5 min. at room temperature. The layers were separated. The aqueous layer was extracted with CH₂Cl₂ and the organic layer was washed with H₂O, dried (MgSO₄), filtered off and the solvent was evaporated. The residue was purified by column chromatography over silica gel (eluent gradient: CH₂Cl₂/CH₃OH from 100/0 to 90/10). The product fractions were collected and the solvent was evaporated. Yield: 2.1 g of final compound 7 (50 %).

### b) Preparation of final compound 8

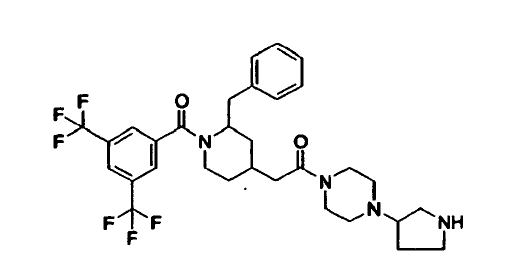

A mixture of final compound 7 (0.00295 mol) in CF₃COOH (20 ml) was stirred at room temperature for 1 hour. The reaction mixture was poured out into ice/H₂O/K₂CO₃. The aqueous layer was extracted with CH₂Cl₂ and the organic layer was dried (MgSO₄), filtered off and the solvent was evaporated. Yield: 1.7 g of final compound 8 (94 %).

### Example B4

### a) Preparation of final compound 9

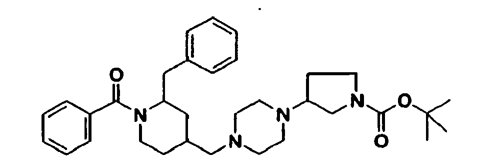

A mixture of intermediate compound 16 (prepared according to A6) (0.0088 mol) and 1,1-dimethylethyl 3-(1-piperazinyl)-1-pyrrolidinecarboxylic acid ester ((prepared according to the teachings in WO2004/056799 of which the content is included herein by reference) (0.01 mol) in methanol (150 ml) was hydrogenated at 50°C for 48 hours with Pd/C 10 % (1 g) as a catalyst in the presence of thiophene solution (1 ml). After uptake of H₂(1 eq.), the catalyst was filtered off and the filtrate was evaporated. The residue was purified by column chromatography over silica gel (eluent gradient: CH₂Cl₂/CH₃OH from 100/0 to 95/5). The product fractions were collected and the solvent was evaporated. Yield: 3.6 g of final compound 9 (75 %).

### b) Preparation of final compound 10

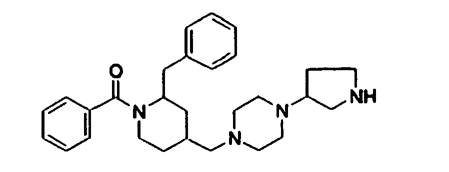

A mixture of final compound 9 (prepared according to B4.a) (0.0066 mol) in HCl/2-propanol (10 ml) and methanol (40 ml) was stirred and refluxed for 1 hour. The solvent was evaporated and the residue was taken up in H₂O. The mixture was alkalised with NaOH and extracted with CH₂Cl₂. The organic layer was washed with H₂O, dried (MgSO₄), filtered off and the solvent was evaporated. Yield: 2.6 g of final compound 10 (88 %).

### Example B5

### Preparation of final compound 11

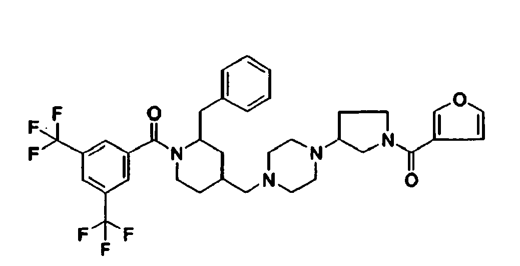

A mixture of 3-furancarboxylic acid (0.0027 mol), DMAP (0.0027 mol) and Et₃N (0.0027 mol) in CH₂Cl₂, p.a. (100 ml) was stirred at room temperature. Then, EDCI (0.0027 mol) was added and the reaction mixture was stirred at room temperature for 1 hour, giving mixture (I). A mixture of final compound 60 (prepared according to B 1.d) (0.002 mol) dissolved in CH₂Cl₂ was added dropwise to mixture (I) and the reaction mixture was stirred overnight at room temperature. The mixture was poured out into NaOH (1 N) and stirred for 15 min. at room temperature. The layers were separated. The aqueous layer was extracted with CH₂Cl₂ and the organic layer was dried (MgSO₄), filtered off and the solvent was evaporated. The residue was purified by column chromatography over silica gel (eluent gradient: CH₂Cl₂/CH₃OH from 100/0 to 90/10). The product fractions were collected and the solvent was evaporated. Yield: 0.914 g of final compound 11 (67 %)

### Example B6

### Preparation of final compound 12

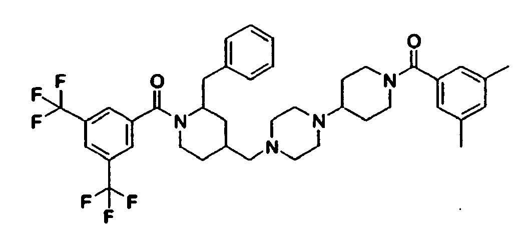

A mixture of final compound 6 (prepared according to B1.d) (0.0021 mol) and Et₃N (0.01 mol) in CH₂Cl₂, p.a. (50 ml) was stirred on an ice-bath. 3.5-dimethylbenzoyl chloride (0.0025 mol) was dissolved in CH₂Cl₂ and added dropwise to the reaction mixture which was stirred for 1 hour at room temperature. K₂CO₃ (2 g) was added and H₂O and the mixture was extracted. The separated aqueous layer was extracted with CH₂Cl₂ and the separated combined organic layers were dried (MgSO₄), filtered and the filtrate was evaporated. The residue was purified by column chromatography over silica gel (eluent gradient: CH₂Cl₂/MeOH from 100/0 to 90/10). The product fractions were collected and the solvent was evaporated. Yield: 1.38 g of final compound 12 (90 %).

### Example B7

### Preparation of final compound 13

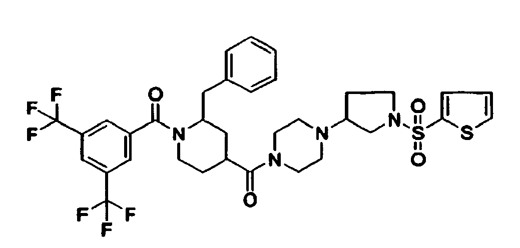

A mixture of final compound 66 (prepared according to B1.d) (0.00134 mol) and Et₃N (0.01 mol) in CH₂Cl₂, p.a. (40 ml) was stirred at room temperature, then a solution of 2-thiophenesulfonyl chloride (0.0016 mol) in CH₂Cl₂, p.a. (q.s.) was added dropwise and the reaction mixture was stirred overnight at room temperature. NaOH (1 N, 20 ml) was added dropwise, the resulting mixture was stirred for 10 min. at room temperature and then the layers were separated. The aqueous layer was extracted with CH₂Cl₂ ; the organic layer was washed with H₂O, dried (MgSO₄), filtered off and the solvent was evaporated. The residue was purified by column chromatography over silica gel (eluent gradient: CH₂Cl₂/CH₃OH from 100/0 to 90/10). The product fractions were collected and the solvent was evaporated. Yield: 0.791 g of final compound 13 (80 %).

### Example B8

### Preparation of final compound 14

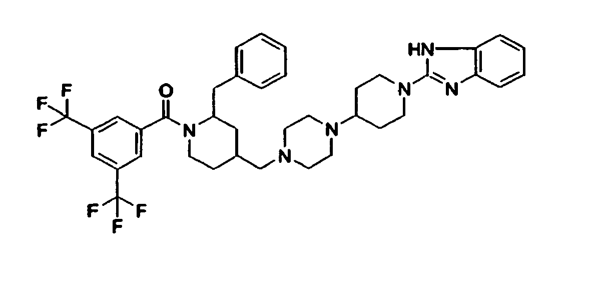

Final compound 6 (prepared according to B2.b) (0.000168 mol) was converted into the free base. A mixture of the free base of final compound 6 (0.000168 mol), 2-chloro-1*H-*benzimidazole (0.000182 mol) and CuO (0.010 g) in *N,N*-dimethylacetamide (3 ml) was stirred for 3 hours at 150°C and then the solvent was evaporated. The obtained residue was taken up in CH₂Cl₂ (5 ml) and H₂O/NH₃ was added. The mixture was stirred for 2 hours and was filtered through Extrelut, then the filter residue was washed with CH₂Cl₂ (2 x 2 ml) and purified by high-performance liquid chromatography (eluent: CH₂Cl₂/CH₃OH 90/10). The product fractions were collected and the solvent was evaporated. Yield: 0.011 g of final compound 14.

### Example B9

### Preparation of final compound 15

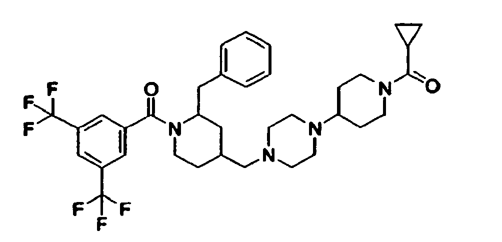

Final compound 6 (prepared according to B2.b) (0.000168 mol) was converted into the free base. A mixture of the free base of final compound 6 (0.000168 mol), cyclopropanecarbonyl chloride (0.001182 mol) and Et₃N (0.0005 mol) in CH₂Cl₂ (4 ml) was stirred over the weekend at room temperature and then H₂O (2 ml) was added. The mixture was stirred for 30 min. and was filtered through Extrelut, then the filter residue was washed with CH₂Cl₂ (2 x 2 ml) and purified by high-performance liquid chromatography (eluent: CH₂Cl₂/CH₃OH 90/10). The product fractions were collected and the solvent was evaporated. Yield: 0.109 g of final compound 15.

### Example B10

### Preparation of final compound 16

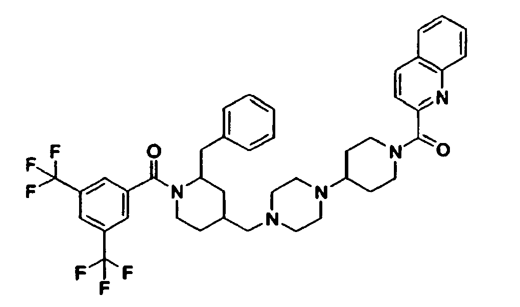

Final compound 6 (prepared according to B2.b) (0.000168 mol) was converted into the free base. A mixture of 2-quinolinecarboxylic acid (0.000182 mol), Et₃N (0.0005 mol) and PyBOP (benzotriazole-1-yl-oxy-tris-pyrrolidino-phosphonium hexafluorophosphate) (0.0002 mol) in CH₂Cl₂ (4 ml) was stirred for 3 hours, then the free base of final compound 6 (0.000168 mol) was added and the reaction mixture was stirred over the weekend at room temperature. The solvent was evaporated and the residue was purified by reversed-phase high-performance liquid chromatography. The product fractions were collected and the solvent was evaporated. Yield: 0.083 g of final compound 16.

### Example B11

### Preparation of final compound 17

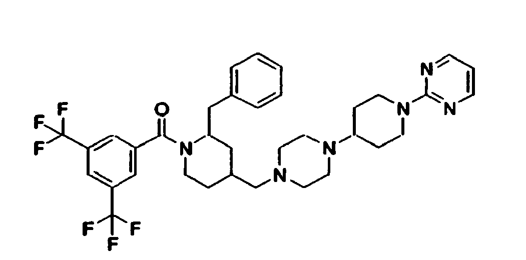

Final compound 6 (prepared according to B2.b) (0.000168 mol) was converted into the free base. A mixture of the free base of final compound 6 (0.000168 mol), 2-chloropyrimidine (0.000182 mol) and Na₂CO₃ (0.0005 mol) in *N*,*N*-dimethylacetamide (4 ml) was stirred over the weekend at 60 °C and then the solvent was evaporated. The obtained residue was taken up in CH₂Cl₂ (5 ml) and H₂O (2 ml) was added. The mixture was stirred for 30 min. and was filtered through Extrelut, then the filter residue was washed with CH₂Cl₂ (2 x 2 ml) and purified by high-performance liquid chromatography (gradient 90/10). The product fractions were collected and the solvent was evaporated. Yield: 0.059 g of final compound 17.

**Table 1**

| | | | | | | | |
|---|---|---|---|---|---|---|---|
| 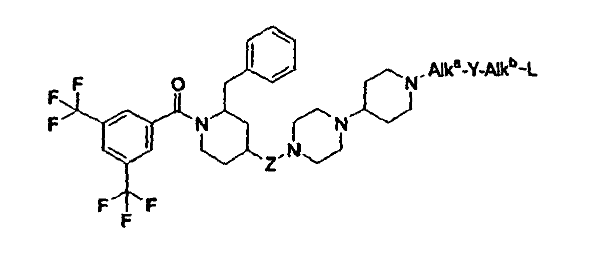 | | | | | | | |

| **Comp. No.** | **Exp. No.** | **Z** | **Alk^{a}** | **Y** | **Alk^{b}** | **L** | **Stereo descriptors** |
|---|---|---|---|---|---|---|---|
| **6** | B2.b | -CH₂- | cb | cb | cb | H | trans; |
| | | | | | | | .HCl(1:3) |
| | | | | | | | .H₂O(1:2) |
| **100** | B2.b | -CH₂- | cb | cb | cb | H | 2S-trans |
| **17** | B11 | -CH₂- | cb | cb | cb | 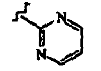 | trans |
| **18** | B8 | -CH₂- | cb | cb | cb | 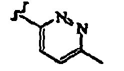 | trans |
| **19** | B8 | -CH₂- | cb | cb | cb | 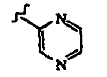 | trans |
| **14** | B8 | -CH₂- | cb | cb | cb | 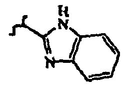 | trans |
| **20** | B11 | -CH₂- | -CH₂- | cb | cb | 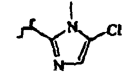 | trans |
| **21** | B11 | -CH₂- | -CH₂- | cb | cb | 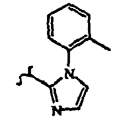 | trans |
| **22** | B11 | -CH₂- | -CH₂- | cb | cb | 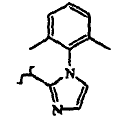 | trans |
| **23** | B11 | -CH₂- | -CHr | cb | cb | 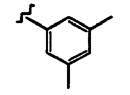 | trans |
| **24** | B11 | -CH₂- | -CH₂- | cb | cb | 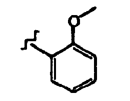 | trans |
| **25** | B11 | -CH₂- | -CH₂- | cb | cb | 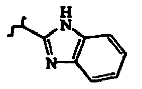 | trans |
| **26** | B11 | -CH₂- | -CHr | cb | cb | 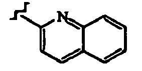 | trans |
| **27** | B11 | -CH₂- | 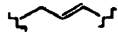 | cb | cb | 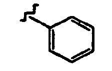 | trans |
| **28** | B11 | -CH₂- | 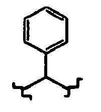 | cb | cb | 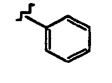 | trans |
| **29** | B11 | -CH₂- | 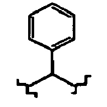 | cb | cb | 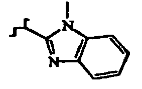 | trans |
| **30** | B11 | -CH₂- | -CH₂- | C=O | cb | 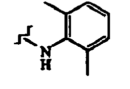 | trans |
| **5** | B2.a | -CH₂- | cb | C=O | cb | 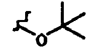 | trans |
| **99** | B2.a | -CH₂- | cb | C=O | cb | 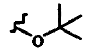 | 2S-trans |
| **31** | B9 | -CH₂- | cb | C=O | cb | 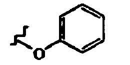 | trans |
| **32** | B6 | -CH₂- | cb | C=O | cb | 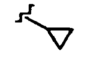 | 2S-trans |
| **15** | B9 | -CH₂- | cb | C=O | cb | 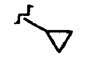 | trans |
| **33** | B10 | -CH₂- | cb | C=O | cb | 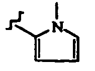 | trans |
| **34** | B6 | -CH₂- | cb | C=O | cb | 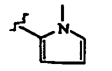 | 2S-trans |
| **35** | B9 | -CH₂- | cb | C=O | cb | 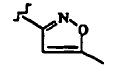 | trans |
| **36** | B6 | -CH₂- | cb | C=O | cb | 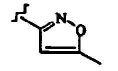 | 2S-trans |
| **37** | B9 | -CH₂- | cb | C=O | cb | 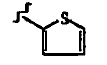 | trans |
| **38** | B10 | -CH₂- | cb | C=O | cb | 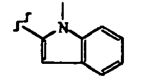 | trans |
| **39** | B10 | -CH₂- | cb | C=O | cb | 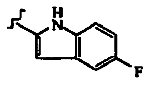 | trans |
| **40** | B10 | -CH₂- | cb | C=O | cb | 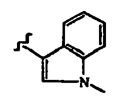 | trans |
| **41** | B6 | -CH₂- | cb | C=O | cb | 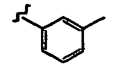 | 2S-trans |
| **12** | B6 | -CH₂- | cb | C=O | cb | 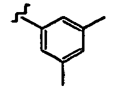 | trans |
| **42** | B9 | -CH₂- | cb | C=O | cb | 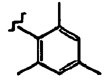 | trans |
| **43** | B10 | -CH₂- | cb | C=O | cb | 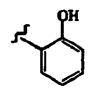 | trans |
| **44** | B10 | -CH₂- | cb | C=O | cb | 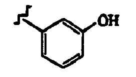 | trans |
| **45** | B9 | -CH₂- | cb | C=O | cb | 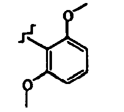 | trans |
| **46** | B9 | -CH₂- | cb | C=O | cb | 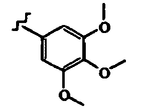 | trans |
| **47** | B9 | -CH₂- | cb | C=O | cb | 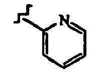 | 2S-trans |
| **48** | B6 | -CH₂- | cb | C=O | cb | 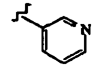 | 2S-trans |
| **49** | B9 | -CH₂- | cb | C=O | cb | 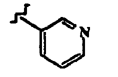 | trans |
| **16** | B10 | -CH₂- | cb | C=O | cb | 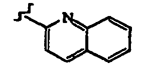 | trans |
| **50** | B10 | -CH₂- | cb | C=O | cb | 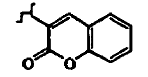 | trans |
| **51** | B10 | -CH₂- | cb | C=O | -CHr | 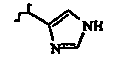 | trans |
| **52** | B9 | -CH₂- | cb | C=O | -CH₂- | 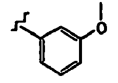 | trans |
| **53** | B10 | -CH₂- | cb | C=O | -CH₂- | 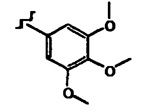 | trans |
| **54** | B9 | -CH₂- | cb | C=O | 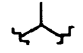 | 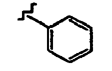 | trans |
| **55** | B10 | -CH₂- | cb | C=O | 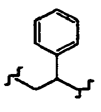 | 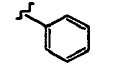 | trans |
| **4** | B1.d | 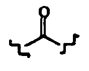 | cb | cb | cb | H | 2S-trans |
| **3** | B1.c | 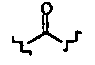 | -CH₂- | cb | cb | 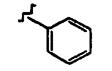 | 2S-trans |
| **56** | B6 | 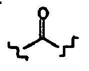 | cb | C=O | cb | 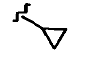 | 2S-trans |
| **92** | B5 | 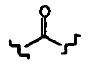 | cb | C=O | cb | 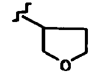 | 2S-trans |
| **57** | B5 | 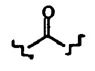 | cb | C=O | cb | 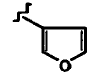 | 2S-trans |
| **58** | B6 | 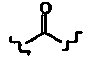 | cb | C=O | cb | 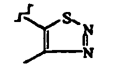 | 2S-trans |
| **59** | B6 | 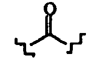 | cb | C=O | cb | 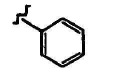 | 2S-trans |

| | | | | | | | |
|---|---|---|---|---|---|---|---|
| cb = covalent bond | | | | | | | |

**Table 2**

| | | | | | | | |
|---|---|---|---|---|---|---|---|
| 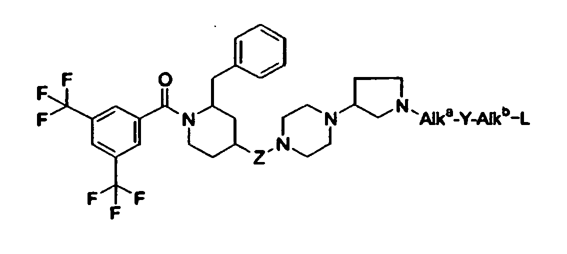 | | | | | | | |

| **Comp. No.** | **Exp. No.** | **Z** | **Alk^{a}** | **Y** | **Alk^{b}** | **L** | **Stereo descriptors** |
|---|---|---|---|---|---|---|---|
| **60** | B1.d | -CH₂- | cb | cb | cb | H | trans |
| **1** | B1.a | -CH₂- | cb | cb | cb | 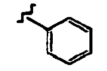 | 2S-trans |
| **61** | B6 | -CH₂- | cb | C=O | cb | 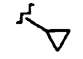 | 2S-trans |
| **11** | B5 | -CH₂- | cb | C=O | cb | 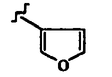 | 2S-trans |
| **62** | B5 | -CH₂- | cb | C=O | cb | 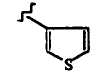 | 2S-trans |
| **63** | B6 | -CH₂- | cb | C=O | cb | 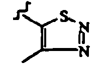 | 2S-trans |
| **64** | B6 | -CH₂- | cb | C=O | cb | 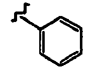 | 2S-trans |
| **65** | B6 | -CH₂- | cb | C=O | cb | 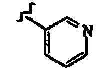 | 2S-trans |
| **66** | B1.d | 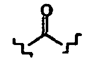 | cb | cb | cb | H | 2S-trans |
| **67** | B1.a | 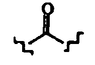 | -CH₂- | cb | cb | 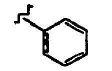 | 2S-trans |
| **68** | B6 | 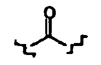 | cb | C=O | cb | 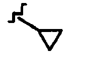 | 2S-trans |
| **69** | B5 | 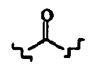 | cb | C=O | cb | 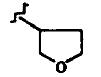 | 2S-trans |
| **70** | B5 | 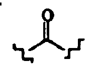 | cb | C=O | cb | 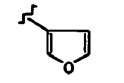 | 2S-trans |
| **71** | B6 | 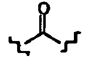 | cb | C=O | cb | 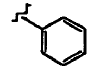 | 2S-trans |
| **72** | B6 | 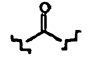 | cb | C=O | cb | 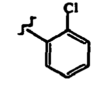 | 2S-trans |
| **13** | B7 | 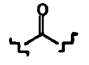 | cb | 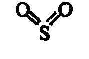 | cb | 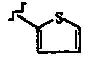 | 2S-trans |
| **8** | B3.b | 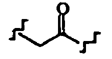 | cb | cb | cb | H | 2R cis/trans |
| **7** | B3.a | 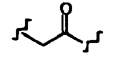 | cb | C=O | cb | 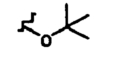 | 2R cis/trans |
| **73** | B5 | 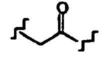 | cb | C=O | cb | 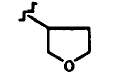 | 2R cis/trans |
| **74** | B6 | 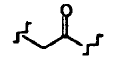 | cb | C=O | cb | 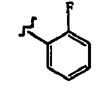 | 2R cis/trans |
| **75** | B5 | 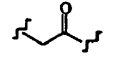 | cb | C=O | cb | 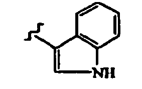 | 2R cis/trans |
| **94** | B3.b | 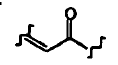 | cb | cb | cb | H | 2R E/Z |
| **83** | B3.a | 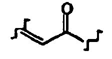 | cb | C=O | cb | 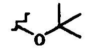 | 2R E/Z |
| **98** | B5 | 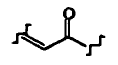 | cb | C=O | cb | 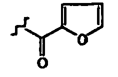 | 2R E/Z |
| **95** | B5 | 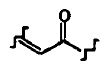 | cb | C=O | cb | 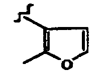 | 2RE/Z |
| **96** | B5 | 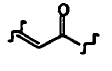 | cb | C=O | cb | 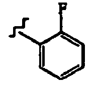 | 2R E/Z |
| **97** | B5 | 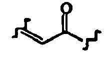 | cb | C=O | cb | 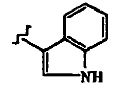 | 2R E/Z |

| | | | | | | | |
|---|---|---|---|---|---|---|---|
| cb = covalent bond | | | | | | | |

Some of the experimental numbers refer to analogs; however the compounds according to this Table 2 were prepared in an analogous way with intermediate compounds as described above.

**Table 3**

| | | | | | | | |
|---|---|---|---|---|---|---|---|
| 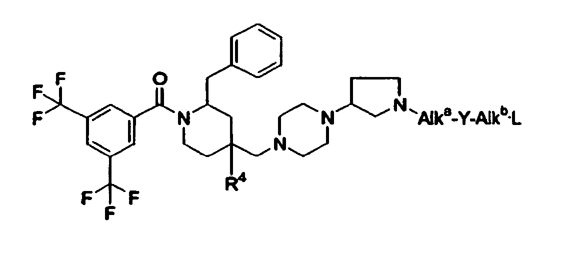 | | | | | | | |

| **Comp. No.** | **Exp. No.** | **R⁴** | **Alk^{a}** | **Y** | **Alk^{b}** | **L** | **Stereo descriptors** |
|---|---|---|---|---|---|---|---|
| **76** | B1.d | -OH | cb | cb | cb | H | 2R-cis |
| **2** | B1.b | -OH | -CH₂- | cb | cb | 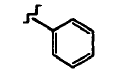 | 2R-cis |
| **77** | B6 | -OH | cb | C=O | cb | 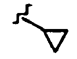 | 2R-cis |
| **78** | B5 | -OH | cb | C=O | cb | 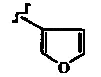 | 2R-cis |
| **79** | B6 | -OH | cb | C=O | cb | 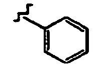 | 2R-cis |
| **80** | B6 | -OH | cb | C=O | cb | 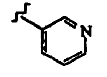 | 2R-cis |
| **81** | B1.d | -OCH₃ | cb | cb | cb | H | 2R-cis |
| **82** | B1.a | -OCH₃ | -CH₂- | cb | cb | 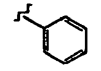 | 2R-cis |
| **84** | B6 | -OCH₃ | cb | C=O | cb | 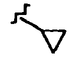 | 2R-cis |
| **85** | B5 | -OCH₃ | cb | C=O | cb | 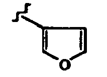 | 2R-cis |
| **86** | B6 | -OCH₃ | cb | C=O | cb | 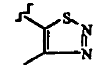 | 2R-cis |
| **93** | B6 | -OH | cb | C=O | cb | 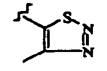 | 2R-cis |
| **87** | B6 | -OCH₃ | cb | C=O | cb | 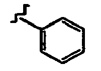 | 2R-cis |
| **88** | B6 | -OCH₃ | cb | C=O | cb | 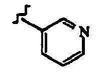 | 2R-cis |
| **89** | B7 | -OCH₃ | cb | 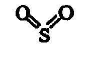 | cb | 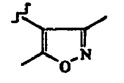 | 2R-cis |

| | | | | | | | |
|---|---|---|---|---|---|---|---|
| cb = covalent bond | | | | | | | |

**Table 4**

| | | | | | | | |
|---|---|---|---|---|---|---|---|
| 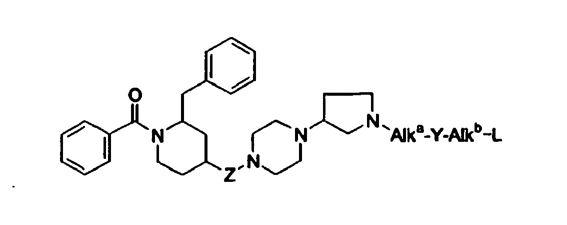 | | | | | | | |

| **Comp. No.** | **Exp. No.** | **Z** | **Alk^{a}** | **Y** | **Alk^{b}** | **L** | **Stereo descriptors** |
|---|---|---|---|---|---|---|---|
| **10** | B4.b | -CH₂- | cb | cb | cb | H | 2S-trans |
| **9** | B4.a | -CH₂- | cb | C=O | cb | 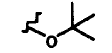 | 2S-trans |
| **90** | B5 | -CH₂- | cb | C=O | cb | 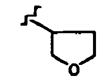 | 2S-trans |
| **91** | B5 | -CH₂- | cb | C=O | cb | 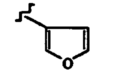 | 2S-trans |

| | | | | | | | |
|---|---|---|---|---|---|---|---|
| cb = covalent bond | | | | | | | |

### C. Analytical data

For a number of compounds, either melting points or LCMS data were recorded.

### 1. LCMS conditions

The HPLC gradient was supplied by a Waters Alliance HT 2790 system with a columnheater set at 40°C. Flow from the column was split to a Waters 996 photodiode array (PDA) detector and a Waters-Micromass ZQ mass spectrometer with an electrospray ionization source operated in positive and negative ionization mode. Reversed phase HPLC was carried out on a Xterra MS C18 column (3.5 µm, 4.6 x 100 mm) with a flow rate of 1.6 ml/min. Three mobile phases (mobile phase A 95% 25mM ammoniumacetate + 5% acetonitrile; mobile phase B: acetonitrile; mobile phase C: methanol) were employed to run a condition from 100 % A to 50 % B and 50 % C in 6.5 min., to 100 % B in 1 min, 100% B for 1 min. and reequilibrate with 100 % A for 1.5 min. An injection volume of 10 µL was used.

Mass spectra were acquired by scanning from 100 to 1000 in 1 s using a dwell time of 0.1 s. The capillary needle voltage was 3 kV and the source temperature was maintained at 140 °C . Nitrogen was used a the nebulizer gas. Cone voltage was 10 V for positive ionzation mode and 20 V for negative ionization mode. Data acquisition was performed with a Waters-Micromass MassLynx-Openlynx data system.

**Table 5 : LCMS parent peak and retention time for selected compounds.**

| **Comp. no.** | **Retention time (minutes)** | **LCMS (MH+)** |
|---|---|---|
| **1** | 8.1 | 673 |
| **2** | 8.42 | 689 |
| **3** | 6.6 | 701 |
| **4** | 5.13 | 611 |
| **5** | 8.21 | 697 |
| **6** | 6.55 | 597 |
| **7** | 7.88 | 711 |
| **8** | 6.3 | 611 |
| **9** | 6.6 | 547 |
| **11** | 7.3 | 677 |
| **12** | 8.1 | 729 |
| **13** | 5.8 | 743 |
| **14** | 7.33 | 713 |
| **15** | 7.3 | 665 |
| **16** | 7.66 | 752 |
| **18** | 7.31 | 689 |
| **19** | 7.71 | 675 |
| **21** | 7.96 | 767 |
| **22** | 8.46 | 781 |
| **23** | 8.71 | 715 |
| **25** | 7.54 | 727 |
| **26** | 8.04 | 738 |
| **27** | 8.19 | 713 |
| **28** | 8.11 | 763 |
| **29** | 8.81 | 817 |
| **30** | 7.84 | 758 |
| **31** | 8.21 | 717 |
| **32** | 7.46 | 665 |
| **33** | 7.56 | 704 |
| **34** | 7.8 | 704 |
| **35** | 7.36 | 706 |
| **36** | 7.53 | 706 |
| **37** | 7.54 | 707 |
| **38** | 8.14 | 754 |
| **39** | 8.07 | 758 |
| **40** | 8.01 | 754 |
| **41** | 7.86 | 715 |
| **42** | 8.23 | 743 |
| **43** | 7.56 | 717 |
| **44** | 7.2 | 717 |
| **45** | 7.51 | 761 |
| **46** | 7.46 | 789 |
| **47** | 7.26 | 702 |
| **48** | 7.01 | 702 |
| **49** | 7 | 702 |
| **50** | 7.54 | 769 |
| **51** | 6.89 | 705 |
| **52** | 7.78 | 745 |
| **53** | 7.54 | 805 |
| **54** | 8.14 | 729 |
| **55** | 8.46 | 805 |
| **56** | 5.8 | 679 |
| **57** | 5.77 | 705 |
| **58** | 5.79 | 737 |
| **59** | 5.99 | 715 |
| **61** | 7.36 | 651 |
| **62** | 7.61 | 693 |
| **63** | 7.36 | 709 |
| **64** | 7.55 | 687 |
| **65** | 7.19 | 688 |
| **66** | 5.2 | 597 |
| **67** | 6.38 | 687 |
| **68** | 5.58 | 665 |
| **69** | 5.42 | 695 |
| **70** | 5.72 | 691 |
| **71** | 6.14 | 701 |
| **72** | 6.24 | 735 |
| **73** | 6.79 | 709 |
| **74** | 7.33 | 733 |
| **75** | 6.13 | 755 |
| **76** | 6.58 | 599 |
| **77** | 7.36 | 667 |
| **78** | 7.29 | 693 |
| **79** | 7.52 | 703 |
| **80** | 6.88 | 704 |
| **81** | 6.78 | 613 |
| **82** | 8.34 | 703 |
| **84** | 7.74 | 683 |
| **85** | 7.64 | 707 |
| **86** | 7.59 | 739 |
| **87** | 7.79 | 717 |
| **88** | 7.25 | 718 |
| **89** | 7.99 | 772 |
| **90** | 4.73 | 545 |
| **91** | 5.07 | 541 |
| **92** | 5.61 | 709 |
| **93** | 7.33 | 725 |

### D. Pharmacological example

### Example D.1 : Binding experiment for h-NK₁, h-NK₂ and h-NK₃ receptors

The compounds according to the invention were investigated for interaction with various neurotransmitter receptors, ion channels and transporter binding sites using the radioligand binding technique. Membranes from tissue homogenates or from cells, expressing the receptor or transporter of interests, were incubated with a radioactively labelled substance ([³H]- or [¹²⁵I] ligand) to label a particular receptor. Specific receptor binding of the radioligand was distinguished from the non-specific membrane labelling by selectively inhibiting the receptor labelling with an unlabelled drug (the blank), known to compete with the radioligand for binding to the receptor sites. Following incubation, labelled membranes were harvested and rinsed with excessive cold buffer to remove non-bound radioactivity by rapid filtration under suction. Membrane bound radioactivity was counted in a scintillation counter and results were expressed in counts per minute (cpm).

The compounds were dissolved in DMSO and tested at 10 concentrations ranging from 10⁻¹⁰ to 10⁻⁵ M.

The ability of the compounds according to the invention to displace [³H]-Substance P from cloned human h-NK₁ receptors expressed in CHO cells, to displace [³H]-SR-48968 from cloned human h-NK₂ receptors expressed in Sf9 cells, and to displace [³H]-SR-142801 from cloned human h-NK₃ receptors expressed in CHO cells was evaluated.

The receptor binding values (pIC₅₀) for the h-NK₁ ranges for all compounds according to the invention between 10 and 6.

### Example D.2 : Signal transduction (ST)

This test evaluates in vitro functional NK₁ antagonistic activity. For the measurements of intracellular Ca⁺⁺ concentrations the cells were grown on 96-well (black wall/transparent bottom) plates from Costar for 2 days until they reached confluence. The cells were loaded with 2 µM Fluo3 in DMEM containing 0.1 % BSA and 2.5 mM probenecid for 1 h at 37°C. They were washed 3x with a Krebs buffer (140 mM NaCl, 1 mM MgCl₂x6H₂O, 5 mM KCl, 10 mM glucose, 5 mM HEPBS; 1.25 mM CaCl₂; pH 7.4) containing 2.5 mM probenecid and 0.1 % BSA (Ca⁺⁺-buffer). The cells were preincubated with a concentration range of antagonists for 20 min at RT and Ca⁺⁺-signals after addition of the agonists were measured in a Fluorescence Image Plate Reader (FLIPR from Molecular Devices, Crawley, England). The peak of the Ca⁺⁺-transient was considered as the relevant signal and the mean values of corresponding wells were analysed as described below.

The sigmoidal dose response curves were analysed by computerised curve-fitting, using the GraphPad Program. The EC₅₀-value of a compound is the effective dose showing 50 % of maximal effect For mean curves the response to the agonist with the highest potency was normalised to 100 %. For antagonist responses the IC₅₀-value was calculated using non-linear regression.

The pIC₅₀ data for the signal transduction testing for a representative selection of compounds are presented in Table 6. The last colums indicates - without being limited thereto - for which use the compounds might be most suitable. Ofcourse, since for some neurokinin receptors no data was determined, it is obvious that these compounds might be attributed to another suitable use.

**Table 6 : Pharmacological data for the signal transduction for selected compounds. (n.d.= not determined)**

| **Comp. No.** | **NK₁ pIC₅₀** | **NK₂ pIC₅₀** | **NK₃ pIC₅₀** | **Suitable for** |
|---|---|---|---|---|
| **40** | 6.46 | n.d. | n.d. | NK1 |
| **3** | 6.46 | n.d. | <5 | NK1 |
| **12** | 6.50 | n.d. | n.d. | NK1 |
| **52** | 6.50 | 5.63 | ~5.01 | NK1 |
| **8** | 6.52 | n.d. | <5 | NK1 |
| **19** | 6.54 | <5 | 5.1 | NK1 |
| **37** | 6.56 | 5.05 | 5.15 | NK1 |
| **4** | 6.60 | n.d. | <5 | NK1 |
| **18** | 6.64 | <5 | <5 | NK1 |
| **46** | 6.64 | n.d. | n.d. | NK1 |
| **33** | 6.65 | n.d. | n.d. | NK1 |
| **66** | 6.67 | n.d. | 5.17 | NK1 |
| **34** | 6.68 | n.d. | n.d. | NK1 |
| **5** | 6.68 | n.d. | <5 | NK1 |
| **47** | 6.69 | n.d. | n.d. | NK1 |
| **81** | 6.70 | n.d. | <5 | NK1 |
| **15** | 6.72 | ~5.5 | ~5.11 | NK1 |
| **60** | 6.74 | <5 | <5 | NK1 |
| **7** | 6.76 | n.d. | <5 | NK1 |
| **41** | 6.78 | n.d. | n.d. | NK1 |
| **76** | 6.83 | <5 | <5 | NK1 |
| **16** | 6.86 | n.d. | n.d. | NK1 |
| **43** | 6.91 | n.d. | n.d. | NK1 |
| **51** | 6.92 | n.d. | n.d. | NK1 |
| **64** | 6.93 | <5 | <5 | NK1 |
| **87** | 6.94 | n.d. | <5 | NK1 |
| **65** | 6.98 | <5 | <5 | NK1 |
| **62** | 7.00 | 5.14 | <5 | NK1 |
| **20** | 7.03 | <5 | <5 | NK1 |
| **32** | 7.04 | n.d. | 5.41 | NK1 |
| **50** | 7.04 | n.d. | n.d. | NK1 |
| **44** | 7.06 | n.d. | n.d. | NK1 |
| **84** | 7.06 | n.d. | <5 | NK1 |
| **17** | 7.07 | 5.14 | 5.28 | NK1 |
| **53** | 7.07 | n.d. | n.d. | NK1 |
| **67** | 7.12 | n.d. | <5 | NK1 |
| **61** | 7.13 | <5 | <5 | NK1 |
| **63** | 7.15 | <5 | <5 | NK1 |
| **86** | 7.17 | n.d. | 4.99 | NK1 |
| **85** | 7.19 | n.d. | <5 | NK1 |
| **88** | 7.21 | n.d. | <5 | NK1 |
| **89** | 7.22 | n.d. | 5.45 | NK1 |
| **74** | 7.25 | n.d. | ~5.45 | NK1 |
| **11** | 7.3 | 5.13 | ~5.1 | NK1 |
| **36** | 7.33 | n.d. | n.d. | NK1 |
| **77** | 7.37 | 5.1 | 5.54 | NK1 |
| **73** | 7.38 | n.d. | <5 | NK1 |
| **93** | 7.4 | 5.11 | 5.37 | NK1 |
| **69** | 7.4 | n.d. | 5.22 | NK1 |
| **78** | 7.44 | 5.05 | 5.6 | NK1 |
| **79** | 7.5 | 5.04 | 5.14 | NK1 |
| **72** | 7.5 | n.d. | <5 | NK1 |
| **80** | 7.52 | 5.09 | <5 | NK1 |
| **48** | 7.59 | <5 | n.d. | NK1 |
| **70** | 7.59 | n.d. | <5 | NK1 |
| **57** | 7.64 | n.d. | 5.61 | NK1 |
| **75** | 7.8 | n.d. | 5.22 | NK1 |
| **68** | 7.88 | n.d. | 5.18 | NK1 |
| **13** | 7.88 | n.d. | 5.60 | NK1 |
| **59** | 7.97 | n.d. | 5.02 | NK1 |
| **58** | 8.06 | n.d. | <5 | NK1 |
| **92** | 7.62 | 5.17 | 6.06 | NK1/NK3 |
| **56** | 7.91 | 5.33 | 6.16 | NK1/NK3 |

### E. Composition examples

"Active ingredient" (A.I.) as used throughout these examples relates to a compound of Formula (I), the pharmaceutically acceptable acid or base addition salts thereof, the stereochemically isomeric forms thereof, the *N*-oxide form thereof and prodrugs thereof.

### Example E.1 : ORAL DROPS

500 Grams of the A.I. was dissolved in 0.51 of 2-hydroxypmpanoic acid and 1.5 1 of the polyethylene glycol at 60~80°C. After cooling to 30~40°C there were added 35 1 of polyethylene glycol and the mixture was stirred well. Then there was added a solution of 1750 grams of sodium saccharin in 2.5 1 of purified water and while stirring there were added 2.5 1 of cocoa flavor and polyethylene glycol q.s. to a volume of 501, providing an oral drop solution comprising 10 mg/ml of A.I. The resulting solution was filled into suitable containers.

### Example E.2 : ORAL SOLUTION

9 Grams of methyl 4-hydroxybenzoate and 1 gram of propyl 4-hydroxybenzoate were dissolved in 41 of boiling purified water. In 3 1 of this solution were dissolved first 10 grams of 2,3-dihydroxybutanedioic acid and thereafter 20 grams of the A.I. The latter solution was combined with the remaining part of the former solution and 12 11,2,3-propanetriol and 3 1 of sorbitol 70% solution were added thereto. 40 Grams of sodium saccharin were dissolved in 0.51 of water and 2 ml of raspberry and 2 ml of gooseberry essence were added. The latter solution was combined with the former, water was added q.s. to a volume of 201 1 providing an oral solution comprising 5 mg of the active ingredient per teaspoonful (5 ml). The resulting solution was filled in suitable containers.

### Example E.3 : FILM-COATED TABLETS

### Preparation of tablet core

A mixture of 100 grams of the A.I., 570 grams lactose and 200 grams starch was mixed well and thereafter humidified with a solution of 5 grams sodium dodecyl sulfate and 10 grams polyvinylpyrrolidone in about 200 ml of water. The wet powder mixture was sieved, dried and sieved again. Then there was added 100 grams microcrystalline cellulose and 15 grams hydrogenated vegetable oil. The whole was mixed well and compressed into tablets, giving 10.000 tablets, each containing 10 mg of the active ingredient

### Coating

To a solution of 10 grams methyl cellulose in 75 ml of denaturated ethanol there was added a solution of 5 grams of ethyl cellulose in 150 ml of dichloromethane. Then there were added 75 ml of dichloromethane and 2.5 ml 1,2,3-propanetriol. 10 Grams of polyethylene glycol was molten and dissolved in 75 ml of dichloromethane. The latter solution was added to the former and then there were added 2.5 grams of magnesium octadecanoate, 5 grams of polyvinylpyrrolidone and 30 ml of concentrated colour suspension and the whole was homogenated. The tablet cores were coated with the thus obtained mixture in a coating apparatus.

### Example E.4 : INJECTABLE SOLUTION

1.8 Grams methyl 4-hydroxybenzoate and 0.2 grams propyl 4-hydroxybenzoate were dissolved in about 0.51 of boiling water for injection. After cooling to about 50°C there were added while stirring 4 grams lactic acid, 0.05 grams propylene glycol and 4 grams of the A.I.. The solution was cooled to room temperature and supplemented with water for injection q.s. ad 11, giving a solution comprising 4 mg/ml of A.I.. The solution was sterilized by filtration and filled in sterile containers.

## Claims

1. A compound according to the general Formula (I) 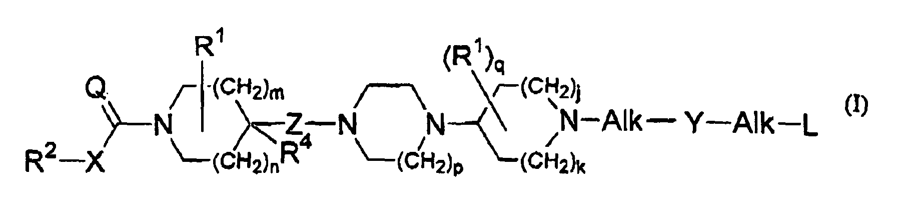 the pharmaceutically acceptable acid or base addition salts thereof, the
stereochemically isomeric forms thereof, and the *N*-oxide form thereof
wherein :
n is an integer, equal to 1 ;
m is an integer, equal to 1;
each R¹ independently from each other, is selected from the group of Ar¹, Ar¹-alkyl and di(Ar¹)-alkyl ;
R⁴ is selected from the group of hydrogen, hydroxy and alkyloxy ;
Z is a bivalent radical -(CH₂)ᵣ, wherein r is an integer equal to 1, 2, 3, 4 or 5 and wherein one radical -CH₂- is optionally replaced by a >C=O radical ; or
R⁴ and Z are taken together to form a trivalent radical =CH-(CH₂)ᵣ₋₁-, wherein r is an integer equal to 2, 3, 4 or 5 and wherein one radical -CH₂- is optionally replaced by a >C=O radical ;
p is an integer, equal to 1 ;
Q is O Or NR³ ;
X is a covalent bond or a bivalent radical of formula -O-, -S- or -NR³- ;
each R³ independently from each other, is hydrogen or alkyl ;
R² is alkyl, Ar², Ar²-alkyl, Het¹ or Het¹-alkyl ;
q is an integer equal to 0 or 1 ;
j is an integer, equal to 1 ;
k is an integer, equal to 0 or 1 ;
Y is a covalent bond or a bivalent radical of formula >C(=O) or -SO₂-;
each Alk represents, independently from each other, a covalent bond ; a bivalent straight or branched, saturated or unsaturated hydrocarbon radical having from 1 to 6 carbon atoms ; or a cyclic saturated or unsaturated hydrocarbon radical having from 3 to 6 carbon atoms ; each radical optionally substituted on one or more carbon atoms with one or more alkyl, phenyl, halo, cyano, hydroxy, formyl and amino radicals ;
L is selected from the group of hydrogen, alkyl, alkyloxy, Ar³-oxy, alkyloxycarbonyl, mono- and di(alkyl)amino, mono- and di(Ar³)amino, mono- and di(alkyloxycarbonyl)amino, Ar³, Ar³-carbonyl, Het² and
Het²-carbonyl ;
Ar¹ is phenyl, optionally substituted with 1, 2 or 3 substituents each independently from each other selected from the group of halo, alkyl, cyano, aminocarbonyl and alkyloxy ;
Ar² is naphthalenyl or phenyl, each optionally substituted with 1, 2 or 3 substituents, each independently from each other, selected from the group of halo, nitro, amino, mono- and di(alkyl)amino, cyano, alkyl, hydroxy, alkyloxy, carboxyl, alkyloxycarbonyl, aminocarbonyl and mono- and di(alkyl)aminocarbonyl ;
Ar³ is naphthalenyl or phenyl, optionally substituted with 1, 2 or 3 substituents each independently from each other selected from the group of alkyloxy, alkyl, halo, hydroxy, pyridinyl, morpholinyl, pyrrolidinyl, imidazo[1,2-*a*]pyridinyl, morpholinylcarbonyl, pyrrolidinylearbonyl, amino and cyano;
Het¹ is a monocyclic heterocyclic radical selected from the group of pyrrolyl, pyrazolyl, imidazolyl, furanyl, thienyl, oxazolyl, isoxazolyl, thiazolyl, thiadiazolyl, isothiazolyl, pyridinyl, pyrimidinyl, pyrazinyl and pyridazinyl ; or a bicyclic heterocyclic radical selected from the group of quinolinyl, quinoxalinyl, indolyl, benzimidazolyl, benzoxazolyl, benzisoxazolyl, benzothiazolyl, benzisothiazolyl, benzofuranyl and benzothienyl ; each heterocyclic radical may optionally be substituted on any atom by one or more radicals selected from the group of halo and alkyl ;
Het² is a monocyclic heterocyclic radical selected from the group of pyrrolidinyl, dioxolyl, imidazolidinyl, pyrrazolidinyl, piperidinyl, morpholinyl, dithianyl, thiomorpholinyl, piperazinyl, imidazolidinyl, tetrahydrofuranyl, 2H-pyrrolyl, pyrrolinyl, imidazolinyl, pyrrazolinyl, pyrrolyl, imidazolyl, pyrazolyl, triazolyl, furanyl, thienyl, oxazolyl, isoxazolyl, thiazolyl, thiadiazolyl, isothiazolyl, pyridinyl, pyrimidinyl, pyrazinyl, pyridazinyl and triazinyl ; or a bicyclic heterocyclic radical selected from the group of benzopiperidinyl, quinolinyl, quinoxalinyl, indolyl, isoindolyl, chromenyl, benzimidazolyl, imidazo[1,2-a]pyridinyl, benzoxazolyl, benzisoxazolyl, benzothiazolyl, benzisothiazolyl, benzofuranyl and benzothienyl ; each heterocyclic radical may optionally be substituted on any atom by one or more radicals selected from the group of Ar¹, Ar¹alkyl, halo, hydroxy, alkyl, piperidinyl, pyrrolyl, thienyl, oxo, alkyloxy, alkyloxyalkyl and alkyloxycarbonyl ; and
alkyl is a straight or branched saturated hydrocarbon radical having from 1 to 6 carbon atoms or a cyclic saturated hydrocarbon radicals having from 3 to 6 carbon atoms ; optionally substituted on one or more carbon atoms with one or more radicals selected from the group of phenyl, halo, cyano, oxo, hydroxy, formyl and amino radicals.

2. A compound according to claim 1, **characterized in that** R¹ is Ar¹ methyl and attached to the 2-position or R¹ is Ar¹ and attached to the 3-position.

3. A compound according to any one of claims 1 to 2, **characterized in that** the R²-X-C(=Q)- moiety is 3,5-di-(trifluoromethyl) phenylcarbonyl.

4. A compound according to any one of claims I to 3, **characterized in that** Z is selected from the group of -CH₂-, >C=O, -CH₂CH₂- and -CH₂C(=O)- or **characterized in that** Z and R⁴ are taken together to form the trivalent radical =CH-C(=O)-.

5. A compound according to any one of claims 1 to 4, **characterized in that** R⁴ is selected from the group of hydrogen, hydroxy and methoxy.

6. A compound according to any one of claims 1 to 5, **characterized in that** Alk is a covalent bond, -CH₂-, CH(CH₃)-, -CH(phenyl)-, -CH₂CH(phenyl)- or -CH₂CH=H-.

7. A compound according to any one of claims 1 to 6, **characterized in that** L is selected from the group of hydrogen, alkyl, alkyloxy, Ar³-oxy, mono- and di(Ar³)amino, Ar³, Het² and Het²carbonyl and Ar³ and Het² are defined as in Formula(I).

8. A compound according to any one of claims 1 to 7, **characterized in that**
n is an integer, equal to 1 ;
m is an integer, equal to 1 ;
R¹ is Ar¹-alkyl ;
R⁴ is selected from the group of hydrogen, hydroxy or alkyloxy ;
Z is a bivalent radical -(CH₂)ᵣ-, wherein r is 1 or 2 and wherein one radical -CH₂- is optionally replaced by a >C=O radical ; or
R⁴ and Z are taken together to form a trivalent radical =CH-(CH₂)ᵣ₋₁, wherein r is 2 and wherein one radical -CH₂- is replaced by a >C=O radical ;
p is an integer, equal to 1 ;
Q is O;
X is a covalent bond;
R² is Ar² ;
q is an integer, equal to 0 ;
j is an integer, equal to 1 ;
k is an integer, equal to 0 or 1 ;
Y is a covalent bond or a bivalent radical of formula >C(=O) or -SO₂-
each Alk represents, independently from each other, a covalent bond ; a bivalent straight or branched, saturated or unsaturated hydrocarbon radical having from I to 6 carbon atoms ; each radical optionally substituted on one or more carbon atoms with a phenyl radical ;
L is selected from the group of hydrogen, alkyl, alkyloxy, Ar³-oxy, mono- and di(Ar³)amino, Ar³, Het² and Het²carbonyl;
Ar¹ is phenyl ;
Ar² is phenyl, optionally substituted with 2 alkyl substituents ;
Ar³ is phenyl, optionally substituted with 1, 2 or 3 substituents, each independently from each other selected from the group of alkyloxy, alkyl, halo and hydroxy ;
Het² is a monocyclic heterocyclic radical selected from the group of tetrahydrofuranyl, pyrrolyl, imidazolyl, pyrazolyl, furanyl, thienyl, isoxazolyl, thiadiazolyl, pyridinyl, pyrimidinyl, pyrazinyl and pyridazinyl ; or a bicyclic heterocyclic radical selected from the group of quinolinyl, indolyl, chromenyl and benzimidazolyl ; each heterocyclic radical may optionally be substituted on any atom by one or more radicals selected from the group of Ar¹, halo, alkyl and oxo ; and
alkyl is a straight or branched saturated hydrocarbon radical having from 1 to 6 carbon atoms or a cyclic saturated hydrocarbon radicals having from 3 to 6 carbon atoms.

9. A compound according to any one of claims 1 to 8 for use as a medicine.

10. The use of a compound according to any one of claims 1 to 8 for the manufacture of a medicament for treating neurokinin mediated conditions.

11. The use of a compound according to claim 10 for the manufacture of a medicament for the treatment and/or prophylaxis of schizophrenia, emesis, anxiety and depression, irritable bowel syndrome (IBS), circadian rhythm disturbances, pre-eclampsia, nociception, pain, in particular visceral and neuropathic pain, pancreatitis, neurogenic inflammation, asthma, chronic obstructive pulmonary disease (COPD) and micturition disorders such as urinary incontinence.

12. A pharmaceutical composition comprising a pharmaceutically acceptable carrier and, as active ingredient, a therapeutically effective amount of a compound according to any one of claims 1 to 8.

13. A pharmaceutical composition according to claim 12, **characterized in that** it is in a form suitable to be orally administered.

14. A process for the preparation of a pharmaceutical composition according to any one of claims 12 to 13, **characterized in that** a pharmaceutically acceptable carrier is intimately mixed with a therapeutically effective amount of a compound as claimed in any one of claims 1 to 8.

15. A process for the preparation of a compound according to Formula (I), more specifically according to Formula (I^{a}), Formula (I^{b}) or Formula (I^{c}), **characterized in that**
a) a final compound according to Formula (I) is obtained by reductive *N-*alkylation of an intermediate according to Formula (II) wherein wherein R¹, R², R⁴, X, Q, m, n, p and Z are defined as in Formula (I), with a *N*-substituted piperidinon of Formula (III) wherein R¹, Alk, Y, L, J, k and q are defined as in Formula (I), in a reaction-inert solvent and in the presence of a reducing agent ; or 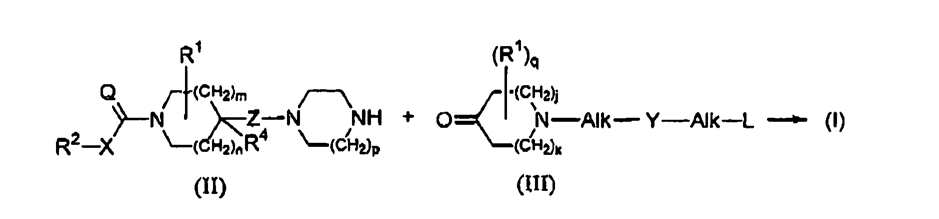
b) a final compound according to Formula (I^{a}) is obtained by reacting of a final compound of Formula (I') wherein R¹, R², R⁴, X, Q, Z, m, n, p and q are defined as in Formula (I), with an acyl compound of Formula (V) wherein Alk and L are defined as in Formula (I) and W¹ is a leaving group, in a reaction-inert solvent and in the presence of a base ; or 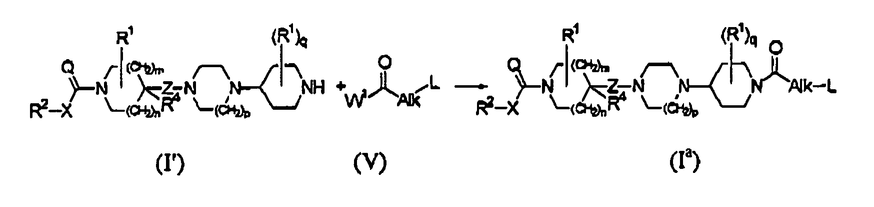
c) a final compound according to Formula (I^{a}) is obtained by a base-catalyzed nucleophilic addition reaction of a final compound of Formula (I') wherein R¹, R², R⁴, X, Q, Z, m, n, p and q are defined as in Formula (I), with a carboxylic acid of Formula (VI) wherein Alk and L are defined as in Formula (I), in a reaction-inert solvent and in the presence of a base; or 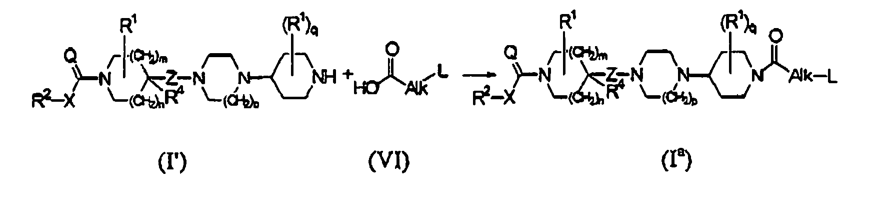
d) a final compound according to Formula (I^{b}) is obtained by a base-catalyzed nucleophilic addition reaction of a final compound of Formula (I') wherein R¹, R², R⁴, X, Q, Z, m, n, p and q are defined as in Formula (I), with a keto-compound of Formula (VII) wherein Alk and L are defined as in Formula (I) and W² is a leaving group, in a reaction-inert solvent and in the presence of a base ; or 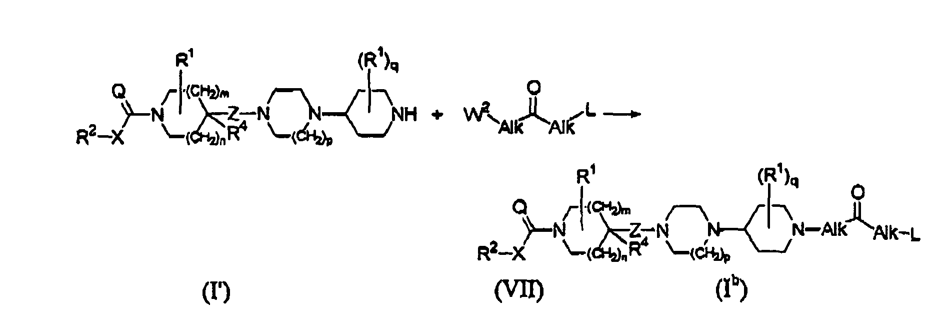
e) a final compound according to Formula (1°) is obtained by reductive amination/alkylation of a final compound of Formula (I') wherein R¹, R², X, Q, m, n, p and q are defined as in Formula (I) with a compound of Formula (VIII) wherein Alk and L are defined as in Formula (I) and W³ is a leaving group, in a reaction-inert solvent and in the presence of a base ; or 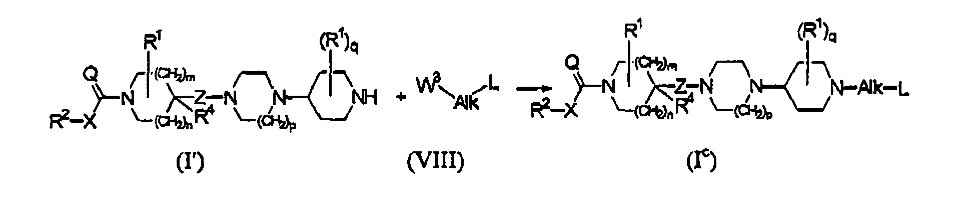
f) a final compound according to Formula (I) is obtained by converting compounds according to Formula (I) into each other following art-known transformation reactions ; and further, optionally converting compounds according to Formula (I) into an acid addition salt by treatment with an acid, or into a base addition salt by treatment with a base, or conversely, the acid addition salt form may be converted into the free base by treatment with alkali, or the base addition salt may be converted into the free acid by treatment with an acid ; and by preparing the
*N*-oxide and/or stereochemically isomeric forms thereof.

## Patentansprüche

1. Verbindungen der allgemeinen Formel (I) 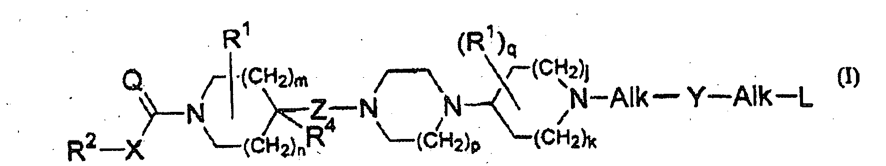 deren pharmazeutisch unbedenkliche Säure- oder Basenadditionssalze, deren stereochemisch isomere Formen und deren N-Oxidformen, wobei:
n für eine ganze Zahl gleich 1 steht;
m für eine ganze Zahl gleich 1 steht;
R¹ jeweils unabhängig von den anderen aus der aus Ar¹, Ar¹-Alkyl und Di(Ar¹)-alkyl bestehenden Gruppe ausgewählt ist;
R⁴ ausgewählt ist aus der Gruppe bestehend aus Wasserstoff, Hydroxy und Alkyloxy;
Z für einen zweiwertigen Rest -(CH₂)ᵣ- steht, wobei r für eine ganze Zahl gleich 1, 2, 3, 4 oder 5 steht und wobei ein Rest -CH₂- gegebenenfalls durch einen >C=O-Rest ersetzt ist; oder
R⁴ und Z zusammen einen dreiwertigen Rest =CH-(CH₂)ᵣ₋₁-bilden, wobei r für eine ganze Zahl gleich 2, 3, 4 oder 5 steht und wobei ein Rest -CH₂-gegebenenfalls durch einen >C=O-Rest ersetzt ist;
p für eine ganze Zahl gleich 1 steht;
Q für O oder NR³ steht;
X für eine kovalente Bindung oder einen zweiwertigen Rest der Formel -O-, -S- oder -NR³-steht;
R³ jeweils unabhängig von den anderen für Wasserstoff oder Alkyl steht;
R² für Alkyl, Ar², Ar²-Alkyl, Het¹ oder Het¹-Alkyl steht;
q für eine ganze Zahl gleich 0 oder 1 steht;
j für eine ganze Zahl gleich 1 steht;
k für eine ganze Zahl gleich 0 oder 1 steht;
Y für eine kovalente Bindung oder einen zweiwertigen Rest der Formel >C(=O)- oder -SO₂-steht;
Alk jeweils unabhängig von den anderen für eine kovalente Bindung; einen zweiwertigen geradkettigen oder verzweigten, gesättigten oder ungesättigten Kohlenwasserstoffrest mit 1 bis 6 Kohlenstoffatomen; oder einen cyclischen gesättigten oder ungesättigten Kohlenwasserstoffrest mit 3 bis 6 Kohlenstoffatomen steht;
wobei die Reste jeweils gegebenenfalls an einem oder mehreren Kohlenstoffatomen durch einen oder mehrere Alkyl-, Phenyl-, Halogen-, Cyano-, Hydroxy-, Formyl- oder Aminoreste substituiert sind;
L aus der aus Wasserstoff, Alkyl, Alkyloxy, Ar³-Oxy, Alkyloxycarbonyl, Mono- und Di(alkyl)amino, Mono- und Di(Ar³)amino, Mono-und Di(alkyloxycarbonyl)amino, Ar³, Ar³-Carbonyl, Het² und Het²-Carbonyl bestehenden Gruppe ausgewählt ist;
Ar¹ für Phenyl steht, gegebenenfalls substituiert durch 1, 2 oder 3 Substituenten jeweils unabhängig voneinander ausgewählt aus der aus Halogen, Alkyl, Cyano, Aminocarbonyl und Alkyloxy bestehenden Gruppe;
Ar² für Naphthalinyl oder Phenyl steht, jeweils gegebenenfalls substituiert durch 1, 2 oder 3 Substituenten jeweils unabhängig voneinander ausgewählt aus der aus Halogen, Nitro, Amino, Mono- und Di(alkyl)amino, Cyano, Alkyl, Hydroxy, Alkyloxy, Carboxyl, Alkyloxycarbonyl, Aminocarbonyl und Mono- und Di(alkyl)aminocarbonyl bestehenden Gruppe;
Ar³ für Naphthalinyl oder Phenyl steht, gegebenenfalls substituiert durch 1, 2 oder 3 Substituenten jeweils unabhängig voneinander ausgewählt aus der aus Alkyloxy, Alkyl, Halogen, Hydroxy, Pyridinyl, Morpholinyl, Pyrrolidinyl, Imidazo[1,2-a]pyridinyl, Morpholinylcarbonyl, Pyrrolidinylcarbonyl, Amino und Cyano bestehenden Gruppe;
Het¹ für einen monocyclischen heterocyclischen Rest ausgewählt aus der aus Pyrrolyl, Pyrazolyl, Imidazolyl, Furanyl, Thienyl, Oxazolyl, Isoxazolyl, Thiazolyl, Thiadiazolyl, Isothiazolyl, Pyridinyl, Pyrimidinyl, Pyrazinyl und Pyridazinyl bestehenden Gruppe; oder einen bicyclischen heterocyclischen Rest ausgewählt aus der aus Chinolinyl, Chinoxalinyl, Indolyl, Benzimidazolyl, Benzoxazolyl, Benzisoxazolyl, Benzothiazolyl, Benzisothiazolyl, Benzofuranyl und Benzothienyl bestehenden Gruppe steht;
wobei die heterocyclischen Reste jeweils gegebenenfalls an einem beliebigen Atom durch einen oder mehrere Reste ausgewählt aus der aus Halogen und Alkyl bestehenden Gruppe substituiert sein können;
Het² für einen monocyclischen heterocyclischen Rest ausgewählt aus der aus Pyrrolidinyl, Dioxolyl, Imidazolidinyl, Pyrazolidinyl, Piperidinyl, Morpholinyl, Dithianyl, Thiomorpholinyl, Piperazinyl, Imidazolidinyl, Tetrahydrofuranyl, 2H-Pyrrolyl, Pyrrolinyl, Imidazolinyl, Pyrazolinyl, Pyrrolyl, Imidazolyl, Pyrazolyl, Triazolyl, Furanyl, Thienyl, Oxazolyl, Isoxazolyl, Thiazolyl, Thiadiazolyl, Isothiazolyl, Pyridinyl, Pyrimidinyl, Pyrazinyl, Pyridazinyl und Triazinyl bestehenden Gruppe; oder einen bicyclischen heterocyclischen Rest ausgewählt aus der aus Benzopiperidinyl, Chinolinyl, Chinoxalinyl, Indolyl, Isoindolyl, Chromenyl, Benzimidazolyl, Imidazo[1,2-a]pyridinyl, Benzoxazolyl, Benzisoxazolyl, Benzothiazolyl, Benzisothiazolyl, Benzofuranyl und Benzothienyl bestehenden Gruppe steht; wobei die heterocyclischen Reste jeweils gegebenenfalls an einem beliebigen Atom durch eine oder mehrere Reste ausgewählt aus der aus Ar¹, Ar¹-Alkyl, Halogen, Hydroxy, Alkyl, Piperidinyl, Pyrrolyl, Thienyl, Oxo, Alkyloxy, Alkyloxyalkyl und Alkyloxycarbonyl bestehenden Gruppe substituiert sein können; und
Alkyl für einen geradkettigen oder verzweigten gesättigten Kohlenwasserstoffrest mit 1 bis 6 Kohlenstoffatomen oder einen cyclischen gesättigten Kohlenwasserstoffrest mit 3 bis 6 Kohlenstoffatomen steht; gegebenenfalls an einem oder mehreren Kohlenstoffatomen substituiert durch einen oder mehrere Reste ausgewählt aus der aus Phenyl, Halogen, Cyano, Oxo, Hydroxy, Formyl und Aminoresten bestehenden Gruppe.

2. Verbindungen nach Anspruch 1, **dadurch gekennzeichnet, daß** R¹ für Ar¹-Methyl steht und in der 2-Stellung gebunden ist oder R¹ für Ar¹ steht und in der 3-Stellung gebunden ist.

3. Verbindungen nach Anspruch 1 oder 2, **dadurch gekennzeichnet, daß** es sich bei der R²-X-C(=Q)-Einheit um 3,5-Di(trifluormethyl)phenylcarbonyl handelt.

4. Verbindungen nach einem der Ansprüche 1 bis 3, **dadurch gekennzeichnet, daß** Z ausgewählt ist aus der Gruppe bestehend aus -CH₂-, >C=O, -CH₂CH₂- und -CH₂C(=O)- oder **dadurch gekennzeichnet, daß** Z und R⁴ zusammen den dreiwertigen Rest =CH-C(=O)-bilden.

5. Verbindungen nach einem der Ansprüche 1 bis 4, **dadurch gekennzeichnet, daß** R⁴ ausgewählt ist aus der Gruppe bestehend aus Wasserstoff, Hydroxy und Methoxy.

6. Verbindungen nach einem der Ansprüche 1 bis 5, **dadurch gekennzeichnet, daß** Alk für eine kovalente Bindung, -CH₂-, CH(CH₃)-, -CH(Phenyl)-, -CH₂CH(Phenyl)- oder -CH₂CH=CH- steht.

7. Verbindungen nach einem der Ansprüche 1 bis 6, **dadurch gekennzeichnet, daß** L ausgewählt ist aus der Gruppe bestehend aus Wasserstoff, Alkyl, Alkyloxy, Ar³-Oxy, Mono- und Di(Ar³)amino, Ar³, Het² und Het²-Carbonyl und Ar³ und Het² wie in Formel (I) definiert sind.

8. Verbindungen nach einem der Ansprüche 1 bis 7, **dadurch gekennzeichnet, daß**
n für eine ganze Zahl gleich 1 steht;
m für eine ganze Zahl gleich 1 steht;
R¹ für Ar¹-Alkyl steht;
R⁴ ausgewählt ist aus der aus Wasserstoff, Hydroxy und Alkoxy bestehenden Gruppe;
Z für einen zweiwertigen Rest- (CH₂)ᵣ- steht, wobei r für 1 oder 2 steht und wobei ein Rest -CH₂- gegebenenfalls durch einen Rest >C=O ersetzt ist; oder
R⁴ und Z zusammen einen dreiwertigen Rest =CH-(CH₂)ᵣ₋₁ bilden, wobei r für 2 steht und wobei ein Rest -CH₂- durch einen Rest <C=O ersetzt ist;
p für eine ganze Zahl gleich 1 steht;
Q für O steht;
X für eine kovalente Bindung steht;
R² für Ar² steht;
q für eine ganze Zahl gleich 0 steht;
j für eine ganze Zahl gleich 1 steht;
k für eine ganze Zahl gleich 0 oder 1 steht;
Y für eine kovalente Bindung oder einen zweiwertigen Rest der Formel >C(=O)- oder -SO₂- steht;
Alk jeweils unabhängig von den anderen für eine kovalente Bindung; einen zweiwertigen geradkettigen oder verzweigten, gesättigten oder ungesättigten Kohlenwasserstoffrest mit 1 bis 6 Kohlenstoffatomen steht; wobei die Reste jeweils gegebenenfalls an einem oder mehreren Kohlenstoffatomen durch einen Phenylrest substituiert sind;
L aus der aus Wasserstoff, Alkyl, Alkyloxy, Ar³-Oxy, Mono- und Di(Ar³)amino, Ar³, Het² und Het²-Carbonyl bestehenden Gruppe ausgewählt ist;
Ar¹ für Phenyl steht;
Ar² für gegebenenfalls durch 2 Alkylsubstituenten substituiertes Phenyl steht;
Ar³ für gegebenenfalls durch 1, 2 oder 3 Substituenten jeweils unabhängig voneinander ausgewählt, aus der aus Alkyloxy, Alkyl, Halogen und Hydroxy bestehenden Gruppe substituiertes Phenyl steht;
Het² für einen monocyclischen heterocyclischen Rest ausgewählt aus der aus Tetrahydrofuranyl, Pyrrolyl, Imidazolyl, Pyrazolyl, Furanyl, Thienyl, Isoxazolyl, Thiadiazolyl, Pyridinyl, Pyrimidinyl, Pyrazinyl und Pyridazinyl bestehenden Gruppe steht; oder für einen bicyclischen heterocyclischen Rest ausgewählt aus der aus Chinolinyl, Indolyl, Chromenyl und Benzimidazolyl bestehenden Gruppe steht; wobei die heterocyclischen Reste jeweils gegebenenfalls an einem beliebigen Atom durch einen oder mehrere Reste ausgewählt aus der Gruppe bestehend aus Ar¹, Halogen, Alkyl und Oxo substituiert sein können; und
Alkyl für einen geradkettigen oder verzweigten gesättigten Kohlenwasserstoffrest mit 1 bis 6 Kohlenstoffatomen oder einen cyclischen gesättigten Kohlenwasserstoffrest mit 3 bis 6 Kohlenstoffatomen steht.

9. Verbindungen nach einem der Ansprüche 1 bis 8 zur Verwendung als Medizin.

10. Verwendung einer Verbindung nach einem der Ansprüche 1 bis 8 zur Herstellung eines Medikaments zur Behandlung von durch Neurokinin vermittelten Leiden.

11. Verwendung einer Verbindung nach. Anspruch 10 zur Herstellung eines Medikaments zur Behandlung und/oder Prophylaxe von Schizophrenie, Emesis, Angst und Depression, Reizkolon (Irritable Bowel Syndrome, IBS), Störungen des Tagesrhythmus, Präeclampsie, Nociception, Schmerzen, insbesondere visceralen und neuropathischen Schmerzen, Pankreatitis, neurogener Entzündung, Asthma, chronischer obstruktiver Atemwegerkrankung (Chronic Obstructive Pulmonary Disease, COPD) und Mikturitionsstörungen wie Harninkontinenz.

12. Pharmazeutische Zusammensetzung, enthaltend einen pharmazeutisch unbedenklichen Träger und, als Wirkstoff, eine therapeutisch wirksame Menge einer Verbindung nach einem der Ansprüche 1 bis 8.

13. Pharmazeutische. Zusammensetzung nach Anspruch 12, **dadurch gekennzeichnet, daß** sie in einer für die orale Verabreichung geeigneten Form vorliegt.

14. Verfahren zur Herstellung einer pharmazeutischen Zusammensetzung nach Anspruch 12 oder 13, **dadurch gekennzeichnet, daß** man einen pharmazeutisch unbedenklichen Träger innig mit einer therapeutisch wirksamen Menge einer Verbindung nach einem der Ansprüche 1 bis 8 mischt.

15. Verfahren zur Herstellung einer Verbindung gemäß Formel (I), insbesondere gemäß Formel (I^{a}), Formel (I^{b}) oder Formel (I^{c}), **dadurch gekennzeichnet, daß**
a) man ein Endprodukt gemäß Formel (I) durch reduktive N-Alkylierung eines Zwischenprodukts gemäß Formel (II) , in welchem R¹, R², R⁴, X, Q, m, n, p und Z wie in Formel (I) definiert sind, mit einem N-substituierten Piperidinon der Formel (III), in welchem R¹, Alk, Y, L, j, k und q wie in Formel (I) definiert sind, in einem reaktionsinerten Lösungsmittel und in Gegenwart eines Reduktionsmittels erhält; oder 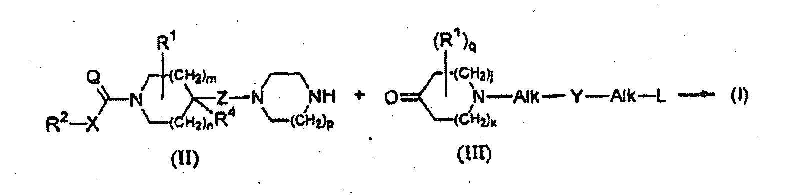
b) man ein Endprodukt gemäß Formel (I^{a}) durch Umsetzung eines Endprodukts der Formel (I'), in welchem R¹, R², R⁴, X, Q, Z, m, n, p und q wie in Formel (I) definiert sind, mit einer Acylverbindung der Formel (V), in welchem Alk und L wie in Formel (I) definiert sind und W¹ für eine Abgangsgruppe steht, in einem reaktionsinerten Lösungsmittel und in Gegenwart einer Base erhält; oder 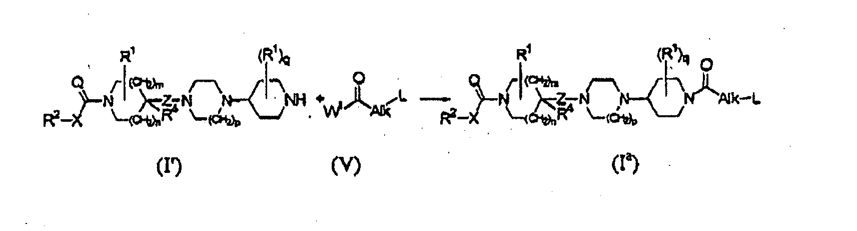
c) man ein Endprodukt gemäß Formel (I^{a}) durch eine basenkatalysierte nukleophile Additionsreaktion eines Endprodukts der Formel (I') in welchem R¹, R², R⁴, X, Q, Z, m, n, p und q wie in Formel (I) definiert sind, mit einer Carbonsäure der Formel (VI), in welcher Alk und L wie in Formel (I) definiert sind, in einem reaktionsinerten Lösungsmittel und in Gegenwart einer Base erhält; oder 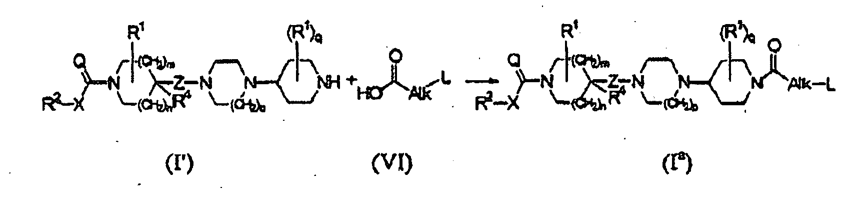
d) man ein Endprodukt gemäß Formel (I^{b}) durch eine basenkatalysierte nukleophile Additionsreaktion eines Endprodukts der Formel (I') in welchem R¹, R², R⁴, X, Q, Z, m, n, p und q wie in Formel (I) definiert sind, mit einer Ketoverbindung der Formel (VII), in welcher Alk und L wie in Formel (I) definiert sind und W² für eine Abgangsgruppe steht, in einem reaktionsinerten Lösungsmittel und in Gegenwart einer Base erhält; oder 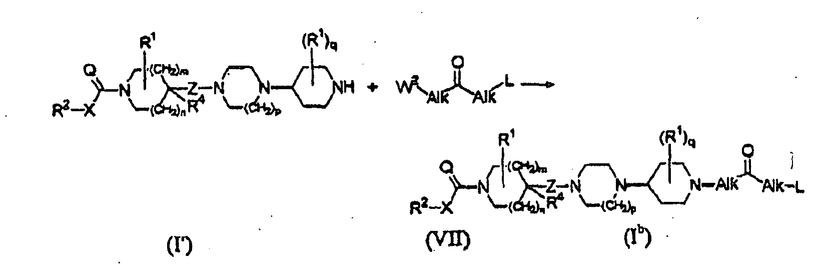
e) man ein Endprodukt gemäß Formel (I^{c}) durch reduktive Aminierung/Alkylierung eines Endprodukts der Formel (I'), in welchem R¹, R², X, Q, m, n, p und q wie in Formel (I) definiert sind, mit einer Verbindung der Formel (VIII), in welcher Alk und L wie in Formel (I) definiert sind und W³ für eine Abgangsgruppe steht, in einem reaktionsinerten Lösungsmittel und in Gegenwart einer Base erhält; oder 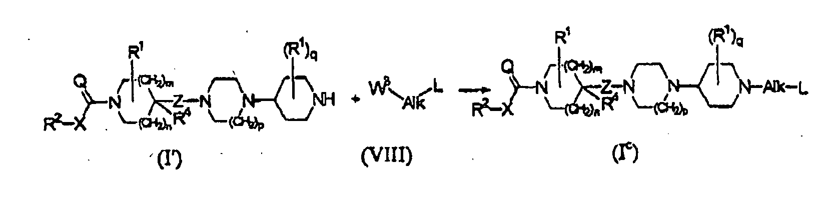
f) man ein Endprodukt gemäß Formel (I) durch Umwandlung von Verbindungen gemäß Formel (I) ineinander nach im Stand der Technik bekannten Transformationsreaktionen erhält; und weiterhin gegebenenfalls Verbindungen gemäß Formel (I) durch Behandlung mit einer Säure in ein Säureadditionssalz umwandelt oder durch Behandlung mit einer Base in ein Basenadditionssalz umwandelt oder umgekehrt die Säureadditionssalzform durch Behandlung mit Alkali in die freie Base umwandeln kann oder das Basenadditionssalz durch Behandlung mit einer Säure in die freie Säure umwandeln kann; und man das N-Oxid- und/oder stereochemisch isomere Formen davon herstellt.

## Revendications

1. Composé selon la formule générale (I) 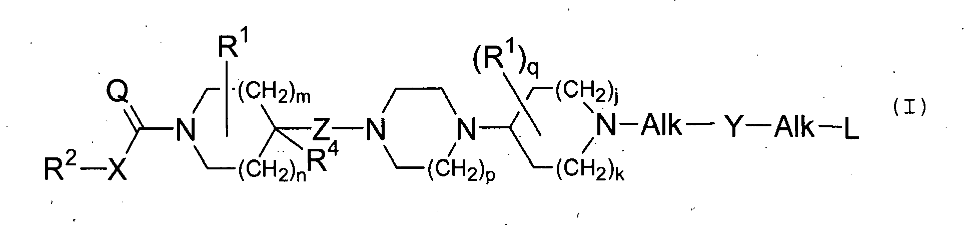 les sels d'addition d'acide ou de base pharmaceutiquement acceptables de celui-ci, les formes stéréochimiquement isomères de celui-ci, et la forme N-oxyde de celui-ci, où :
n est un entier valant 1 ;
m est un entier valant 1 ;
chaque R¹, indépendamment l'un de l'autre, est choisi parmi le groupe constitué d'un groupe Ar¹, d'un groupe Ar¹-alkyle et d'un groupe di(Ar¹)alkyle ;
R⁴ est choisi dans le groupe formé par hydrogène, hydroxy et alkyloxy ;
Z est un radical divalent -(CH₂)ᵣ-, où r est un entier égal à 1, 2, 3, 4 ou 5 et où un radical -CH₂- est éventuellement remplacé par un radical >C=O ; ou
R⁴ et Z sont pris ensemble pour former un radical trivalent =CH-(CH₂)ᵣ₋₁-, où r est un entier égal à 2, 3, 4 ou 5 et où un radical -CH₂- est éventuellement remplacé par un radical >C=O ;
p est un entier valant 1 ;
Q est un atome d'oxygène ou un groupe NR³ ;
X est une liaison covalente ou un radical bivalent de formule -O-, -S- ou -NR³-;
chaque R³, indépendamment l'un de l'autre, est un atome d'hydrogène ou un groupe alkyle ;
R² est un groupe alkyle, un groupe Ar², un groupe Ar²-alkyle, un groupe Het¹ ou un groupe Het¹alkyle ;
q est un entier égal à 0 ou 1 ;
j est un entier égal à 1 ;
k est un entier égal à 0 ou 1 ;
Y est une liaison covalente ou un radical bivalent dé formule >C(=O)- or -SO₂- ;
chaque Alk représente, indépendamment l'un de l'autre, une liaison covalente ; un radical hydrocarboné, saturé ou insaturé, bivalent, linéaire ou ramifié, renfermant de 1 à 6 atomes de carbone ; ou un radical hydrocarboné cyclique, saturé ou insaturé, renfermant de 3 à 6 atomes de carbone ; chaque radical étant éventuellement substitué sur un ou plusieurs atomes de carbone par un ou plusieurs radicaux alkyle phényle, halogéno, cyano, hydroxy, formyle et amino ;
L est choisi parmi le groupe constitué d'un atome d'hydrogène, d'un groupe alkyle, d'un groupe alkyloxy, d'un groupe Ar³-oxy, d'un groupe alkyloxycarbonyle, d'un groupe mono-et di(alkyl)amino, d'un groupe mono- et di (Ar³)amino, d'un groupe mono- et di(alkyloxycarbonyl)amino, d'un groupe Ar³, d'un groupe Ar³-carbonyle, d'un groupe Het² et d'un groupe Het²-carbonyle ;
Ar¹ est un groupe phényle, éventuellement substitué par 1, 2 ou 3 substituants, choisis, chacun indépendamment les uns des autres, parmi le groupe constitué d'un groupe halogéno, d'un groupe alkyle, d'un groupe cyano, d'un groupe aminocarbonyle et d'un groupe alkyloxy ;
Ar² est un groupe naphtalényle ou un groupe phényle, chacun éventuellement substitué par 1, 2 ou 3 substituants choisis, chacun indépendamment les uns des autres, parmi le groupe constitué d'un groupe halogéno, d'un groupe nitro, d'un groupe amino, d'un groupe mono- et di(alkyl)amino, d'un groupe cyano, d'un groupe alkyle, d'un groupe hydroxy, d'un groupe alkyloxy, d'un groupe carboxyle, d'un groupe alkyloxycarbonyle, d'un groupe aminocarbonyle et d'un groupe mono- et di(alkyl)aminocarbonyle ;
Ar³ est un groupe naphtalényle ou un groupe phényle, éventuellement substitué par 1, 2 ou 3 substituants choisis, chacun indépendamment les uns des autres, parmi le groupe constitué d'un groupe alkyloxy, d'un groupe alkyle, d'un groupe halogéno, d'un groupe hydroxy, d'un groupe pyridinyle, d'un groupe morpholinyle, d'un groupe pyrrolidinyle, d'un groupe imidazo[1,2-a]pyridinyle, d'un groupe morpholinylcarbonyle, d'un groupe pyrrolidinylcarbonyle, d'un groupe amino et d'un groupe cyano ;
Het¹ est un radical hétérocyclique monocyclique choisi parmi le groupe constitué d'un groupe pyrrolyle, d'un groupe pyrazolyle, d'un groupe imidazolyle, d'un groupe furanyle, d'un groupe thiényle, d'un groupe oxazolyle, d'un groupe isoxazolyle, d'un groupe thiazolyle, d'un groupe thiadiazolyle, d'un groupe isothiazolyle, d'un groupe pyridinyle, d'un groupe pyrimidinyle, d'un groupe pyrazinyle et d'un groupe pyridazinyle ; ou un radical hétérocyclique bicyclique choisi parmi le groupe constitué d'un groupe quinoléinyle, d'une groupe quinoxalinyle, d'un groupe indolyle, d'un groupe benzimidazolyle, d'un groupe benzoxazolyle, d'un groupe benzisoxazolyle, d'un groupe benzothiazolyle, d'un groupe benzisothiazolyle, d'un groupe benzofuranyle et d'un groupe benzothiényle ; chaque radical hétérocyclique peut être éventuellement substitué sur un atome quelconque par un ou plusieurs radicaux choisis parmi le groupe constitué d'un groupe halogéno et d'un groupe alkyle ;
Het² est un radical hétérocyclique monocyclique choisi parmi le groupe constitué d'un groupe pyrrolidinyle, d'un groupe dioxolyle, d'un groupe imidazolidinyle, d'un groupe pyrrazolidinyle, d'un groupe pipéridinyle, d'un groupe morpholinyle, d'un groupe dithianyle, d'un groupe thiomorpholinyle, d'un groupe pipérazinyle, d'un groupe imidazolidinyle, d'un groupe tétrahydrofuranyle, d'un groupe 2H-pyrrolyle, d'un groupe pyrrolinyle, d'un groupe imidazolinyle, d'un groupe pyrrazolinyle, d'un groupe pyrrolyle, d'un groupe imidazolyle, d'un groupe pyrazolyle, d'un groupe triazolyle, d'un groupe furanyle, d'un groupe thiényle, d'un groupe oxazolyle, d'un groupe isoxazolyle, d'un groupe thiazolyle, d'un groupe thiadiazolyle, d'un groupe isothiazolyle, d'un groupe pyridinyle, d'un groupe pyrimidinyle, d'un groupe pyrazinyle, d'un groupe pyridazinyle et d'un groupe triazinyle ; ou un radical hétérocyclique bicyclique choisi parmi le groupe constitué d'un groupe benzo-pipéridinyle, d'un groupe quinoléinyle, d'un groupe quinoxalinyle, d'un groupe indolyle, d'un groupe isoindolyle, d'un groupe chroményle, d'un groupe benzimidazolyle, d'un groupe imidazo[1,2-a]pyridinyle, d'un groupe benzoxazolyle, d'un groupe benzisoxazolyle, d'un groupe benzothiazolyle, d'un groupe benzisothiazolyle, d'un groupe benzofuranyle et d'un groupe benzothiényle ; chaque radical hétérocyclique peut être éventuellement substitué sur un atome quelconque par un ou plusieurs radicaux choisis parmi le groupe constitué d'un groupe Ar¹, d'un groupe Ar¹-alkyle, d'un groupe halogéno, d'un groupe hydroxy, d'un groupe alkyle, d'un groupe pipéridinyle, d'un groupe pyrrolyle, d'un groupe thiényle, d'un groupe oxo, d'un groupe alkyloxy, d'un groupe alkyloxyalkyle et d'un groupe alkyloxy-carbonyle ;et
alkyle est un radical hydrocarboné saturé, linéaire ou ramifié, renfermant de 1 à 6 atomes de carbone ou un radical hydrocarboné saturé cyclique, renfermant de 3 à 6 atomes de carbone ; éventuellement substitué sur un ou plusieurs atomes de carbone par un ou plusieurs radicaux choisis parmi le groupe constitué, des radicaux phényle, halogéno, cyano, oxo, hydroxy, formyle et amino.

2. Composé selon la revendication 1, **caractérisé en ce que** R¹ représente Ar¹méthyle et est attaché en 2ème position ou R¹ représente Ar¹ et est attaché en 3ème position.

3. Composé selon l'une quelconque des revendications 1 et 2, **caractérisé en ce que** la moitié R²-X-C(=Q)- est un radical 3,5-di-(trifluorométhyl)phénylcarbonyle.

4. Composé selon l'une quelconque des revendications 1 à 3, **caractérisé en ce que** Z est choisi dans le groupe formé par -CH₂-, >C=O, -CH₂CH₂- et -CH₂C(=O)- ou **caractérisé en ce que** Z et R⁴ sont pris ensemble pour former le radical trivalent =CH-C(=O)-.

5. Composé selon l'une quelconque des revendications 1 à 4, **caractérisé en ce que** R⁴ est choisi dans le groupe formé par hydrogène, hydroxy et méthoxy.

6. Composé selon l'une quelconque des revendications 1 à 5, **caractérisé en ce que** Alk est une liaison covalente, -CH₂-, -CH(CH₃)-, -CH(phényl)-, -CH₂CH(phényl)- ou -CH₂CH=CH-.

7. Composé selon l'une quelconque des revendications 1 à 6, **caractérisé en ce que** L est choisi dans le groupe formé par hydrogène, alkyle, alkyloxy, Ar³-oxy, mono (Ar³) amino et di (Ar³) amino, Ar³, Het² et Het²carbonyle et Ar³ et Het² sont définis comme dans la formule (I).

8. Composé selon l'une quelconque des revendications 1 à 7, **caractérisé en ce que**
n représente un entier égal à 1 ;
m représente un entier égal à 1 ;
R¹ représente Ar¹-alkyle ;
R⁴ est choisi dans le groupe formé par hydrogène, hydroxy ou alkyloxy ;
Z est un radical divalent -(CH₂)ᵣ-, où r représente 1 ou 2 et où un radical -CH₂- est éventuellement remplacé par un radical >C=O ; ou
R⁴ et Z sont pris ensemble pour former un radical trivalent =CH-(CH₂)ᵣ₋₁, où r représente 2 et où un radical -CH₂- est éventuellement remplacé par un radical >C=O ;
p représente un entier égal à 1 ;
Q représente O ;
X représente une liaison covalente ;
R² représente Ar² ;
q représente un entier égal à 0 ;
j représente un entier égal à 1 ;
k représente un entier égal à 0 ou 1 ;
Y est une liaison covalente ou un radical bivalent de formule <C(=O)- or -SO₂- ;
chaque Alk représente, indépendamment l'un de l'autre, une liaison covalente ; un radical hydrocarboné, saturé ou insaturé, bivalent, linéaire ou ramifié, renfermant de 1 à 6 atomes de carbone ; chaque radical étant éventuellement substitué sur un ou plusieurs atomes de carbone par un radical phényl ;
L est choisi parmi le groupe constitué d'un atome d'hydrogène, d'un groupe alkyle, d'un groupe alkyloxy, d'un groupe Ar³-oxy, d'un groupe mono- et di(Ar³)amino, d'un groupe Ar³, d'un groupe Het² et d'un groupe Het²-carbonyle ;
Ar¹ est un groupe phényle ;
Ar² est un groupe phényle, éventuellement substitué par 2 substituants alkyle ;
Ar³ est un groupe phényle, éventuellement substitué par 1, 2 ou 3 substituants choisis, chacun indépendamment les uns des autres, parmi le groupe constitué d'un groupe alkyloxy, d'un groupe alkyle, d'un groupe halogéno et d'un groupe hydroxy ;
Het² est un radical hétérocyclique monocyclique choisi parmi le groupe constitué d'un groupe tétrahydrofuranyle, d'un groupe pyrrolyle, d'un groupe imidazolyle, d'un groupe pyrazolyle, d'un groupe furanyle, d'un groupe thiényle, d'un groupe isoxazolyle, d'un groupe thiadiazolyle, d'un groupe pyridinyle, d'un groupe pyrimidinyle, d'un groupe pyrazinyle et d'un groupe pyridazinyle ; ou un radical hétérocyclique bicyclique choisi parmi le groupe constitué d'un groupe quinoléinyle, d'un groupe indolyle, d'un groupe chroményle et d'un groupe benzimidazolyle ; chaque radical hétérocyclique pouvant éventuellement, être substitué sur un quelconque atome par un ou plusieurs radicaux choisis dans le groupe constitué d'un groupe Ar¹, d'un groupe halogéno, d'un groupe alkyle et d'un groupe oxo ; et
alkyle est un radical hydrocarboné saturé linéaire ou ramifié, renfermant de 1 à 6 atomes de carbone
ou un radical hydrocarboné saturé cyclique, renfermant de 3 à 6 atomes de carbone.

9. Composé selon l'une quelconque des revendications 1 à 8 destiné à une utilisation comme médicament.

10. Utilisation d'un composé selon l'une quelconque des revendications 1 à 8 pour la préparation d'un médicament destiné au traitement d'états provoqués par la neurokinine.

11. Utilisation d'un composé selon la revendication 10 pour la préparation d'un médicament destiné au traitement et/ou à la prophylaxie de la schizophrénie, des vomissements, de l'anxiété et de la dépression, du syndrome de l'intestin irritable *(irritable bowel syndrome -* IBS), des troubles du rythme circadien, de la prééclampsie, de la nociception, de la douleur, en particulier la douleur viscérale et neuropathique, de la pancréatite, de l'inflammation neurogénique, de l'asthme, de la broncho-pneumopathie chronique obstructive (BPCO) et des troubles de la miction tels que l'incontinence urinaire.

12. Composition pharmaceutique comprenant un support pharmaceutiquement acceptable et, comme ingrédient actif, une quantité thérapeutiquement efficace d'un composé selon l'une quelconque des revendications 1 à 8.

13. Composition pharmaceutique selon la revendication 12, **caractérisée en ce qu'**elle se trouve sous une forme appropriée pour être administrée par voie orale.

14. Procédé pour la préparation d'une composition pharmaceutique selon l'une quelconque des revendications 12 et 13, **caractérisé en ce qu'**un support pharmaceutiquement acceptable est mélangé intimement avec une quantité thérapeutiquement efficace d'un composé selon l'une quelconque des revendications 1 à 8.

15. Procédé pour la préparation d'un composé selon la formule (I), plus spécifiquement selon la formule (I^{a}), la formule (I^{b}) ou la formule (I^{c}), **caractérisé en ce que**
a) un composé final selon la formule (I) est obtenu par une N-alkylation réductrice d'un intermédiaire selon la formule (II) où R¹, R², R⁴, X, Q, m, n, p et Z sont définis comme dans la formule (I) avec une pipéridone N-substituée de formule (III), où R¹, Alk, Y, L, j, k et q sont définis comme dans la formule (I) dans un solvant inerte à la réaction et en présence d'un réducteur ;
ou 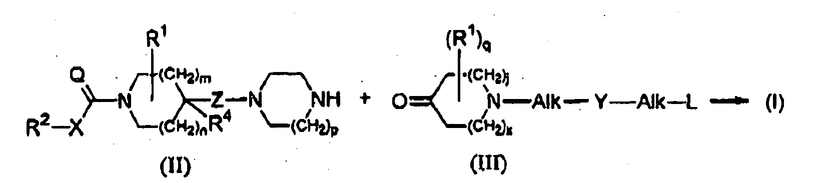
b) un composé final selon la formule (I^{a}) est obtenu en faisant réagir un composé final de formule (I') où R¹, R², R⁴, X, Q, Z, m, n, p et q sont définis comme dans la formule (I) avec un composé acyle de formule (V) où Alk et L sont définis comme dans la formule (I) et W¹ est un groupe partant, dans un solvant inerte à la réaction et en présence d'une base ;
ou 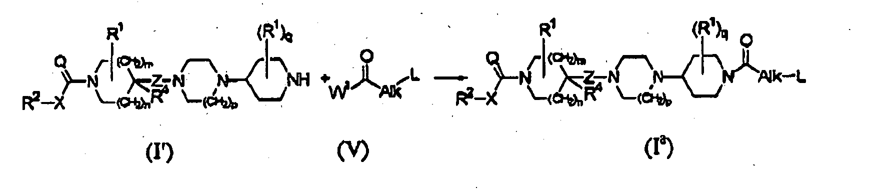
c) un composé final selon la formule (I^{a}) est obtenu par une réaction d'addition nucléophile, catalysée par une base, d'un composé final de formule (I') où R¹, R², R⁴, X, Q, Z, m, n, p et q sont définis comme dans la formule (I) avec un acide carboxylique de formule (VI) où Alk et L sont définis comme dans la formule (I), dans un solvant inerte à la réaction et en présence d'une base ; ou 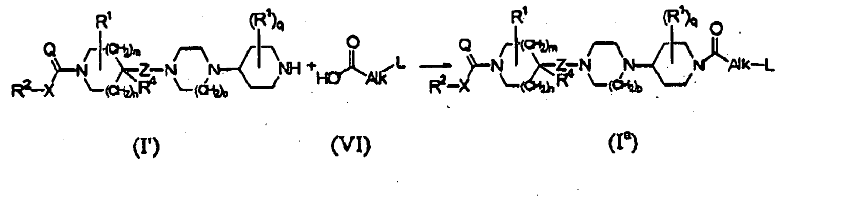
d) un composé final selon la formule (I^{b}) est obtenu par une réaction d'addition nucléophile catalysée par une base, d'un composé final de formule (I') où R¹, R², R⁴, X, Q, Z, m, n, p et q sont définis comme dans la formule (I) avec un composé céto de formule (VII) où Alk et L sont définis comme dans la formule (I) et W² est un groupe partant, dans un solvant inerte à la réaction et en présence d'une base ;
ou 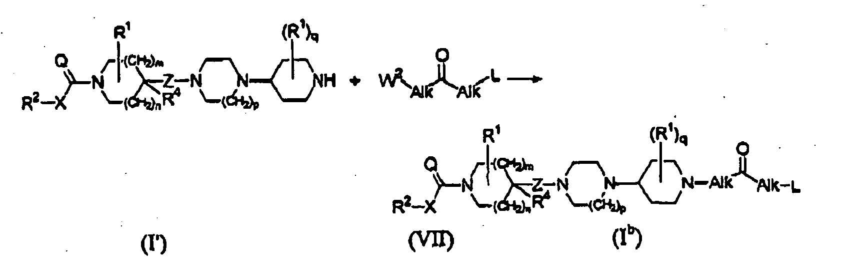
e) un composé final selon la formule (I^{c}) est obtenu par une amination/alkylation réductrice d'un composé final de formule (I') où R¹, R², X, Q, m, n, p et q sont définis comme dans la formule (I) avec un composé de formule (VIII) où Alk et L sont définis comme dans la formule (I) et W³ est un groupe partant, dans un solvant inerte à la réaction et en présence d'une base ; ou 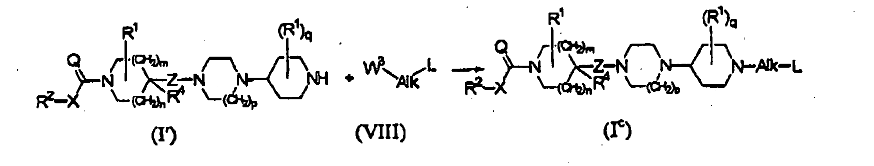
f) un composé final selon la formule (I) est obtenu par conversion de composés selon la formule (I) les uns dans les autres suivant des réactions de transformation connues dans l'état de la technique ; puis, éventuellement les composés selon la formule (I) sont convertis en sel d'addition d'acide par traitement avec un acide ou en sel d'addition de base par traitement avec une base ou, inversement, la forme de sel d'addition d'acide peut être convertie en base libre par traitement avec un alcali ou le sel d'addition de base peut être converti en acide libre par traitement avec un acide ; et par préparation du N-oxyde et/ou des formes stéréochimiquement isomères de celui-ci.
